# FASCICULE DE BREVET EUROPEEN

(11) **EP 4 157 435 B1**
(45) Date de publication et mention de la délivrance du brevet: **20.03.2024**
(21) Numéro de dépôt: 21728548.5
(22) Date de dépôt: 25.05.2021
(51) Int. Cl.: A61N 1/362, A61N 1/365, A61N 1/372, A61N 1/375, G16H 20/30, G16H 40/60

(54) **PROCÉDÉ ET SYSTÈME DE COMMUNICATION ENTRE PLUSIEURS DISPOSITIFS MÉDICAUX IMPLANTABLES**
VERFAHREN UND SYSTEM ZUR KOMMUNIKATION ZWISCHEN EINER VIELZAHL VON IMPLANTIERBAREN MEDIZINISCHEN VORRICHTUNGEN
METHOD AND SYSTEM FOR COMMUNICATION BETWEEN A PLURALITY OF IMPLANTABLE MEDICAL DEVICES

(30) Priorité: 26.05.2020 FR 2005556
(43) Date de publication de la demande: 05.04.2023
(73) Titulaire: Sorin CRM SAS, 92140 Clamart (FR)
(72) Inventeur: MALDARI, Mirko, 75019 Paris (FR)
(74) Mandataire: Ungerer, Olaf
(86) Numéro de dépôt international: PCT/EP2021/063930
(87) Numéro de publication internationale: WO 2021/239741

(56) Documents cités:
- US-A1- 2018 289 973
- US-A1- 2019 247 666
- US-A1- 2019 296 834
- US-B2- 9 687 666

## Description

La présente invention telle que définie dans les revendications 1 et 15 se rapporte à un procédé ainsi qu'à un système de communication entre plusieurs dispositifs médicaux implantables.

Les contractions cardiaques assurent la circulation sanguine. Ces contractions sont produites par un influx électrique appelé stimulus. Dans un coeur sain, le stimulus prend son origine dans le noeud sinusal situé dans la paroi de l'oreillette droite. Issu du noeud sinusal, le stimulus se propage d'abord dans les oreillettes qui vont se contracter pour chasser le sang qu'elles contiennent dans les ventricules encore au repos, et les remplir.

À leur tour, après un délai appelé délai auriculo-ventriculaire, les ventricules sont excités par le stimulus et se contractent. Ce délai auriculo-ventriculaire est essentiel pour un fonctionnement optimal du coeur.

Chez les patients atteints de troubles cardiaques, il arrive que la naissance du stimulus et/ou son cheminement dans l'ensemble du coeur subissent des perturbations à cause de dysfonctions dans le système de conduction du stimulus. Cela cause l'altération du rythme cardiaque, connue comme des arythmies.

Quand le rythme du coeur est trop faible pour satisfaire les besoins d'oxygénation du corps, on parle de bradyarythmie.

Afin de traiter la bradyarythmie, quand les médicaments ne sont pas suffisants, il est connu d'avoir recours à la stimulation artificielle du coeur en utilisant des dispositifs implantables tels que les stimulateurs cardiaques appelés « pacemakers » en anglais. Il est notamment connu d'utiliser un système de stimulation dit stimulateur double chambre (en anglais « double chamber pacemaker »), qui consiste en un dispositif implanté de manière sous-cutanée et connecté à deux sondes qui sont utilisées pour délivrer un stimulus artificiel. Les sondes sont implantées de manière intra-veineuse pour rejoindre l'oreillette droite et le ventricule droit. Lorsque les sondes sont connectées à un même dispositif médical implantable de type stimulateur cardiaque traditionnel, la synchronisation entre les sondes peut être assurée directement par le circuit électronique du dispositif médical implantable. L'utilisation de sondes intra-veineuses présentent toutefois des risques. La fracture de sonde est d'ailleurs l'une des causes les plus courantes de dysfonctionnement du stimulateur cardiaque. L'extraction d'une sonde intraveineuse (ou d'un stimulateur cardiaque) implantée est une procédure à forte morbidité et mortalité élevée, et est donc généralement uniquement effectuée en cas d'infection systémique grave ne pouvant pas être traitée avec des antibiotiques. Dans la majorité des cas, les sondes fracturées seront déconnectées du dispositif et laissées dans le coeur. Une nouvelle sonde est ensuite implantée à côté de l'ancienne et connectée au défibrillateur automatique implantable. Cependant, cette solution n'est possible que s'il reste encore suffisamment d'espace dans la veine, car la présence de davantage de sondes peut entraîner une occlusion veineuse. Par conséquent, l'utilisation de sondes intracardiaques n'est pas idéalement adaptée à de jeunes patients, qui pourraient avoir besoin d'une multitude de sondes au cours de leur vie.

Une solution aux problèmes associés aux sondes intracardiaques, énumérés ci-dessus, consiste à les remplacer par des sondes sous-cutanées et/ou des stimulateurs cardiaques autonomes.

L'un des principaux avantages des stimulateurs cardiaques autonomes est l'absence de boîtier et la réduction des matières étrangères telles que les sondes, ce qui réduit le risque d'infections.

Cependant, les stimulateurs cardiaques autonomes, ne peuvent être utilisés que pour des thérapies à chambre unique, ce qui limite le nombre de patients traitables.

La présente invention se rapporte particulièrement au système de stimulateurs comprenant au moins un stimulateur cardiaque autonome sans sonde, aussi appelé « *leadless cardiac pacemaker »* en anglais. Un stimulateur cardiaque autonome sans sonde se compose d'une source d'énergie telle d'une unité de batterie, de capteurs, d'un générateur de courant et d'un module de télémétrie. Il est notamment conçu pour être implanté à l'intérieur du ventricule droit et peut être utilisé comme alternative aux stimulateurs simple chambre ventriculaire implantables.

Pour augmenter le pourcentage de population qui peut bénéficier d'un stimulateur cardiaque autonome sans sonde, une solution est d'avoir un système de plusieurs dispositifs médicaux implantables, où une communication sans fil entre les dispositifs est nécessaire afin d'envoyer et de recevoir des informations physiologiques telles que la détection des ondes du complexe PQRST. Ces informations sont notamment requises pour pouvoir fournir une thérapie synchronisée. Ainsi, un signal de synchronisation représentant la détection d'une onde du complexe PQRST peut être envoyé par un dispositif implanté dans une chambre pour synchroniser la thérapie d'un autre dispositif implanté dans une autre chambre.

Cependant, les méthodes de communication sans fil pour les dispositifs médicaux implantables, telles que la radiofréquence, la communication intracorporelle (appelée « *intra-body communication (IBC)* » en anglais) et le couplage inductif, sont gourmandes en énergie et réduisent la durée de vie des batteries.

Le problème est d'autant plus critique pour le dispositif qui est configuré pour recevoir le signal de synchronisation car cela nécessite que ses moyens récepteurs restent activés dans l'expectative de la réception d'un signal, réduisant davantage la capacité de la batterie. C'est pourquoi la présente invention a pour objet d'améliorer la communication sans fil de tels systèmes de dispositifs implantable afin de réduire leur consommation en énergie et de prolonger ainsi leur durée de vie. Les documents US 2018/289973 et US 2019/296834 décrivent différents procédés et systèmes de communication entre dispositifs implantables.

L'objet de la présente invention atteint par un procédé de communication dans un système comprenant plusieurs dispositifs médicaux implantables, dans lequel un premier dispositif comprend au moins un moyen de détection d'un signal représentatif de l'activité auriculaire, un moyen émetteur, et un contrôleur configuré pour analyser un signal représentatif de l'activité auriculaire, et un deuxième dispositif, indépendant du premier dispositif médical implantable, comprenant au moins un moyen récepteur et un contrôleur. Le procédé comprend : A) une étape de synchronisation temporelle du premier dispositif avec le second dispositif, par envoi d'un signal de synchronisation du moyen émetteur du premier dispositif vers le deuxième dispositif, le signal de synchronisation étant envoyé suite à l'identification, au moyen du contrôleur du premier dispositif, d'une onde électrique prédéfinie du complexe PQRS d'un signal représentatif de l'activité auriculaire, B) une étape de détermination de la durée d'un cycle cardiaque. Les étapes A et B étant suivies : C) d'une étape de détermination d'un intervalle de synchronisation dont la durée de l'intervalle de synchronisation est déterminée en fonction de la durée du cycle cardiaque de sorte à être plus courte que la durée du cycle cardiaque, et dont le début de l'intervalle de synchronisation est déterminée en fonction du signal de synchronisation, D) d'une étape d'activation du moyen récepteur du deuxième dispositif pendant l'intervalle de synchronisation, le moyen récepteur du deuxième dispositif étant désactivé en dehors de l'intervalle de synchronisation par le contrôleur du deuxième dispositif.

Le procédé de communication est amélioré car le moyen récepteur du deuxième dispositif n'est pas continuellement activé, mais seulement pendant un intervalle de temps plus court que la durée d'un cycle cardiaque.

L'activation partielle du moyen récepteur du deuxième dispositif pendant chaque cycle cardiaque est suffisante aux fins de synchronisation du système, car l'intervalle de synchronisation prend en considération le marqueur temporel qui est relatif à la détection d'une des ondes électriques du complexe PQRS. La détection d'une des ondes électriques du complexe PQRS permet d'identifier un événement cardiaque. L'intervalle de synchronisation est donc déterminé en fonction d'informations physiologiques utiles à la synchronisation du système et relatives au rythme cardiaque du patient. L'intervalle de synchronisation est donc avantageusement ajusté selon des caractéristiques physiologiques du patient.

Le présent procédé permet ainsi de réduire la durée pendant laquelle le moyen récepteur du deuxième dispositif doit être activé pour permettre à la communication sans fil d'avoir lieu. Par conséquent, il est rendu possible d'économiser les besoins énergiques du deuxième dispositif et ainsi d'allonger sa durée de vie.

La présente invention, relative à un procédé de communication, peut être davantage améliorée grâce aux modes de réalisation suivants.

Selon un mode de réalisation, pour un cycle cardiaque, un premier marqueur temporel marquant l'envoi du signal de synchronisation par le moyen émetteur du premier dispositif peut être déterminé, et un deuxième marqueur temporel marquant la réception du signal de synchronisation par le moyen récepteur du deuxième dispositif peut être déterminé, et l'intervalle de synchronisation du premier dispositif pour un cycle cardiaque suivant peut être déterminé en fonction du premier marqueur temporel, l'intervalle de synchronisation du deuxième dispositif pour un cycle cardiaque suivant peut être déterminé en fonction du deuxième marqueur temporel.

Ainsi, l'intervalle de synchronisation du deuxième dispositif est fonction du deuxième marqueur temporel qui est lui-même relatif à une caractéristique physiologique du patient, comme l'onde P, l'onde P étant une des ondes électriques du complexe PQRST.

Selon un mode de réalisation, pendant l'intervalle de synchronisation du deuxième dispositif, le moyen récepteur du deuxième dispositif peut être activé pendant une pluralité de créneaux temporels d'activation prédéfinis et peut être désactivé pendant une pluralité de créneaux temporels de désactivation prédéfinis, la pluralité de créneaux temporels d'activation prédéfinis du deuxième dispositif étant répartie sur l'intervalle de synchronisation du deuxième dispositif de manière à être synchronisée avec des créneaux d'impulsion de signal du premier dispositif en fonction du premier marqueur temporels et du deuxième marqueur temporel.

Ainsi, la consommation d'énergie du moyen récepteur du deuxième dispositif peut être davantage réduite car le moyen récepteur n'est pas activé sur la totalité de l'intervalle de synchronisation mais seulement par créneau. Cela ne gêne en rien la réception d'un signal de synchronisation car les créneaux d'activation sont avantageusement ajustés par rapport au premier marqueur temporel, qui marque l'envoi du signal de synchronisation par le premier dispositif.

Selon un mode de réalisation, le moyen émetteur du premier dispositif peut être configuré pour envoyer un seul signal de synchronisation par cycle cardiaque.

Ainsi, le procédé de communication ne requière pas qu'une série d'impulsion soit émise par le premier dispositif. En effet, le présent procédé permet que le seul signal de synchronisation envoyé par le premier dispositif « tombe » dans un créneau temporel d'activation du moyen récepteur du deuxième dispositif grâce à la synchronisation des premier et deuxième marqueurs temporels.

Selon un mode de réalisation, chacun des créneaux temporels d'activation prédéfinis peut être de même durée et chacun des créneaux temporels de désactivation prédéfinis peut être de même durée et chacun des créneaux temporels d'activation et de désactivation prédéfinis peuvent se succéder alternativement dans l'intervalle de synchronisation.

La succession des créneaux temporels d'activation et de désactivation est ainsi périodique, ce qui permet de favoriser davantage la probabilité qu'un signal de synchronisation soit reçu pendant l'intervalle de synchronisation.

Selon un mode de réalisation, la durée d'un créneau temporel d'activation peut être plus courte ou égale que la durée d'un créneau temporel de désactivation.

Ainsi, la consommation d'énergie du moyen récepteur du deuxième dispositif peut être davantage réduite.

Selon un mode de réalisation, la durée d'un créneau temporel d'activation peut représenter entre 0,3 à 50% de la durée d'un créneau temporel de désactivation, en particulier 5 à 10%.

Ainsi, la consommation d'énergie du moyen récepteur du deuxième dispositif peut être d'autant plus réduite.

Selon un mode de réalisation, la durée d'une impulsion pendant laquelle le moyen émetteur du premier dispositif peut être configuré pour émettre un signal de synchronisation correspond à 25 à 80 % de la durée d'un créneau temporel d'activation, en particulier à 50%.

Ainsi, les créneaux temporels d'activation qui correspondent aux créneaux durant lesquels le moyen récepteur du deuxième dispositif est activé pour « écouter » et détecter un signal de synchronisation sont plus longs que la durée d'émission d'un signal de synchronisation. Ainsi, il y a davantage de chance que la réception du signal de synchronisation ait lieu pendant un créneau temporel d'activation du deuxième dispositif.

Selon un mode de réalisation, le premier marqueur temporel peut marquer le début ou la fin de l'envoi du signal de synchronisation ou un temps prédéterminé pendant l'envoi du signal de synchronisation.

L'ajustement de l'intervalle de synchronisation pour le deuxième dispositif avec celui du premier dispositif peut ainsi être amélioré, car le « timing » du premier marqueur temporel est défini avec davantage de précision.

Selon un mode de réalisation, à l'étape A) l'onde électrique prédéfinie détectée au moyen du contrôleur du premier dispositif peut correspondre à l'onde P du complexe PQRS.

La détection de l'onde P est particulièrement adaptée car elle marque la dépolarisation lors de la contraction des oreillettes et constitue ainsi une information physiologique pertinente pour le délai et la synchronisation auriculo-ventriculaire.

Selon un mode de réalisation, à l'étape A), l'onde électrique prédéfinie peut être détectée au moyen du contrôleur du premier dispositif par l'analyse d'un électrogramme, d'un électrocardiogramme, de données mesurées par un accéléromètre, de donnés mesurées par impédance cardiographie ou de donnés mesurées par un capteur acoustique.

Le procédé peut ainsi être avantageusement mis en oeuvre avec différents moyens de détections.

Selon un mode de réalisation, sur au moins deux cycles cardiaques successifs, le premier dispositif et le deuxième dispositif peuvent communiquer entre eux de manière asynchrone pendant les étapes A et B et le moyen récepteur du deuxième dispositif est activé de manière continue pendant les au moins deux cycles cardiaques successifs.

Ainsi, le premier dispositif et le deuxième dispositif sont configurés pour déterminer chacun de manière indépendante un marqueur temporel afin de déterminer et d'ajuster l'intervalle de synchronisation.

Selon un mode de réalisation, le contrôleur du deuxième dispositif peut être configuré pour désactiver le moyen récepteur du deuxième dispositif pendant le temps restant de l'intervalle de synchronisation du deuxième dispositif après la réception du signal de synchronisation.

Ainsi, la consommation d'énergie du moyen récepteur du deuxième dispositif peut être d'autant plus réduite.

Selon un mode de réalisation, le contrôleur du deuxième dispositif peut être configuré pour envoyer un signal d'alerte par un moyen émetteur du deuxième dispositif à destination d'un moyen récepteur du premier dispositif lorsqu'un signal de synchronisation n'est pas reçu par le deuxième dispositif pendant l'intervalle de synchronisation du deuxième dispositif.

Ainsi, le premier dispositif est alerté que le deuxième dispositif, configuré pour être implanté dans le ventricule droit, n'a pas reçu l'information relative à la détection de l'onde électrique prédéfinie. Le premier dispositif ayant envoyé le signal de synchronisation (qui n'a pas été reçu par le deuxième dispositif), le premier dispositif est capable de constater que le signal a été perdu au cours de son cheminement. Une stimulation locale de l'oreillette droite peut alors être nécessaire.

L'objet de la présente invention est également atteint par un système de plusieurs dispositifs médicaux implantables. Le système comprend un premier dispositif comprenant au moins un moyen de détection, un moyen émetteur, et un contrôleur configuré pour analyser un signal représentatif de l'activité auriculaire. Le système comprend un deuxième dispositif, indépendant du premier dispositif médical implantable, comprenant au moins un moyen récepteur et un contrôleur. Le moyen récepteur du deuxième dispositif est configuré pour être activé et désactivé par le contrôleur du deuxième dispositif. Le contrôleur du premier dispositif et le contrôleur du deuxième dispositif sont configurés pour mettre en oeuvre le procédé selon les modes de réalisation décrits ci-dessus.

Ainsi, le système multi-dispositifs implantables est amélioré car le moyen récepteur du deuxième dispositif n'est pas continuellement activé, mais seulement pendant un intervalle de synchronisation plus court que la durée d'un cycle cardiaque.

L'activation partielle du moyen récepteur du deuxième dispositif pendant chaque cycle cardiaque est suffisante aux fins de synchronisation du système, car l'intervalle de synchronisation prend en considération le marqueur temporel qui est relatif à la détection d'une des ondes électriques du complexe PQRS. La détection d'une des ondes électriques du complexe PQRS permet d'identifier un événement cardiaque. L'intervalle de synchronisation est donc déterminé en fonction d'informations physiologiques utiles à la synchronisation du système et relatives au rythme cardiaque du patient. L'intervalle de synchronisation est donc avantageusement ajusté selon des caractéristiques physiologiques du patient.

Selon un mode de réalisation, le premier dispositif médical implantable peut être un dispositif médical implantable sous-cutané, un enregistreur en boucle d'événement ou un stimulateur cardiaque sans sonde, et le deuxième dispositif médical peut être un stimulateur cardiaque sans sonde.

Le système est ainsi adapté pour une thérapie de synchronisation double-chambre. En particulier, le système est apte pour une thérapie de synchronisation double-chambre avec un stimulateur cardiaque sans sonde implantable dans l'oreillette droite et un stimulateur cardiaque sans sonde implantable dans le ventricule droit.

L'invention et ses avantages seront expliqués plus en détail dans ce qui suit au moyen de modes de réalisation préférés et en s'appuyant notamment sur les figures d'accompagnement suivantes, dans laquelle :
La Figure 1 représente un système multi-dispositifs 10 selon un premier mode de réalisation de la présente invention.
La Figure 2 représente schématiquement un tracé standard d'un complexe PQRST :
   La Figure 3 représente un système multi-dispositifs 40 selon un deuxième mode de réalisation de la présente invention.
   La Figure 4 représente un système multi-dispositifs 60 selon un troisième mode de réalisation de la présente invention.
   La Figure 5 représente un diagramme qui illustre schématiquement le procédé de communication selon un premier mode de réalisation de la présente invention.
   La Figure 6 représente un diagramme qui illustre schématiquement le procédé de communication selon un deuxième mode de réalisation de la présente invention.
   La Figure 7 représente un organigramme qui décrit des étapes d'initialisation du procédé mises en oeuvre par un premier dispositif.
   La Figure 8 représente un organigramme qui décrit des étapes opérationnelles du procédé mises en oeuvre par le premier dispositif.
   La Figure 8a illustre une étape de la l'organigramme représenté à la Figure 8.
   La Figure 9 représente un organigramme qui décrit des étapes d'initialisation du procédé mises en oeuvre par un deuxième dispositif.
   La Figure 10 représente un organigramme qui décrit des étapes opérationnelles du procédé mises en oeuvre par le deuxième dispositif.
   La Figure 10a illustre une étape de la l'organigramme représenté à la Figure 10.
   La Figure 11 représente une autre variante du premier et du deuxième mode de réalisation de la présente invention.

L'invention va maintenant être décrite plus en détail en utilisant des modes de réalisation avantageux d'une manière exemplaire et en référence aux figures. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en oeuvre de la présente invention.

La présente invention se rapporte à un procédé de communication pour un système de plusieurs dispositifs médicaux implantables, ainsi qu'à un tel système.

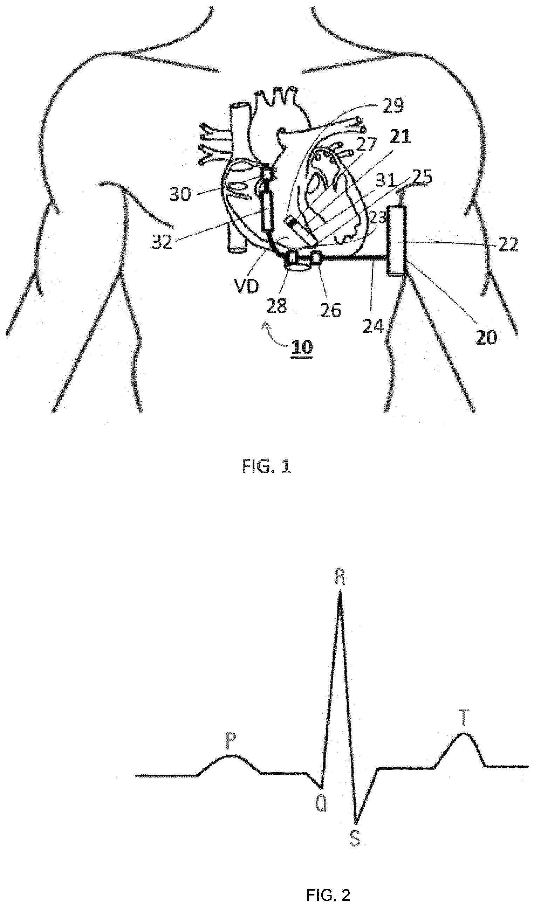

Des modes de réalisation d'un tel système de plusieurs dispositifs médicaux implantables sont d'abord décrits en référence aux Figures 1, 3 et 4.

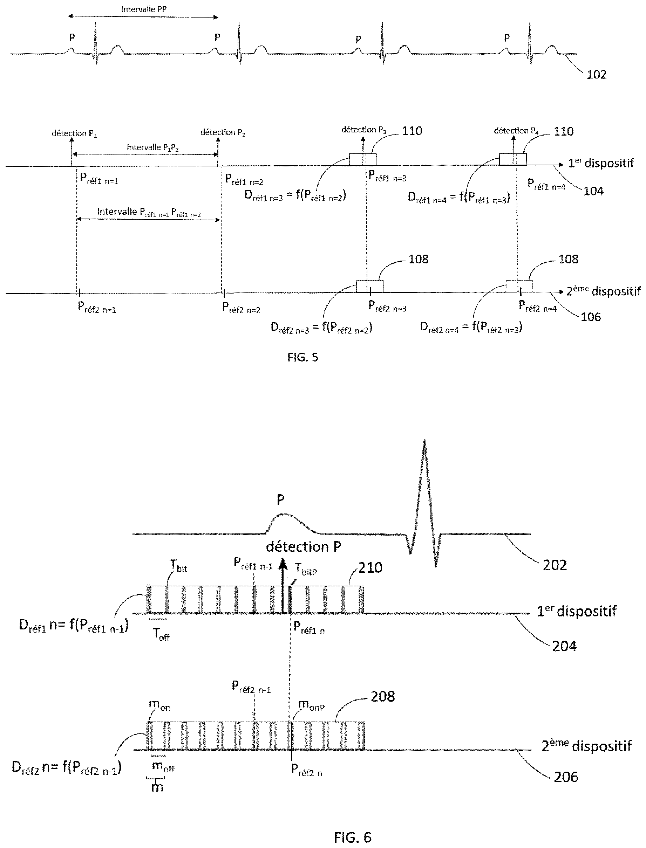

Le procédé de communication selon la présente invention qui peut être mis en oeuvre par un système de plusieurs dispositifs médicaux implantables est ensuite décrit en référence aux Figures 5 à 10.

La Figure 1 représente un système multi-dispositifs 10 selon un premier mode de réalisation de la présente invention comprenant deux dispositifs implantables 20, 21.

Le système multi-dispositifs 10 représenté à la Figure 1 comprend un dispositif implantable sous-cutané 20 et un stimulateur cardiaque autonome sans sonde 21 implanté dans le ventricule droit VD.

Le dispositif implantable sous-cutané 20 représenté à la Figure 1 comprend un boîtier 22 et une sonde sous cutanée 24 pourvue de trois électrodes 26, 28, 30 et d'une électrode de défibrillation 32. Bien que cela ne soit pas visible sur la Figure 1, le boîtier 22 comprend un moyen émetteur, comme un dispositif émetteur radiofréquence (RF) ou utilise une connexion de communication intracorporelle, et un contrôleur. Le boîtier 22 peut également comprendre un moyen récepteur.

Dans une variante, un enregistreur d'événement ou un enregistreur implantable en boucle comprenant au moins un couple d'électrode peut être utilisé à la place du dispositif implantable sous-cutané 20.

Le dispositif implantable sous-cutané 20 est configuré pour détecter l'activité auriculaire en utilisant l'une des méthodes connues dans l'état de l'art, par exemple au moyen d'un électrocardiogramme (ECG), d'une cardiographie d'impédance, d'un détecteur acoustique et/ou d'un accéléromètre.

Dans le premier mode de réalisation de l'invention, le dispositif implantable sous-cutané 20 est configuré pour analyser des données représentatives d'un ECG. L'analyse des données représentatives d'un ECG rend possible la détection d'au moins une des cinq ondes P, Q, R, S, T du complexe PQRST qui sont des caractéristiques électrophysiologiques. Un exemple d'un complexe PQRST est illustré à la Figure 2. Le dispositif implantable sous-cutané 20 est notamment configuré pour identifier un marqueur temporel correspondant à la détection d'une onde électrique du complexe PQRST. La détection de l'onde P est préférée car elle marque la dépolarisation des oreillettes et constitue ainsi une information physiologique pertinente pour le délai et la synchronisation auriculo-ventriculaire.

La détection de l'onde P peut par exemple est déterminé en identifiant un maximum local.

Le stimulateur cardiaque autonome sans sonde 21 comprend une électrode de pointe 23 disposée à une extrémité distale 25 du dispositif 21, et une électrode annulaire 27 disposée vers une extrémité proximale 29 du dispositif 21. Les électrodes 23, 27 peuvent former un dipôle récepteur ou un dipôle émetteur. Notons que la présente invention ne se limite pas à l'utilisation d'une électrode de pointe et d'une électrode de type annulaire mais peut être mise en oeuvre au moyen de tous types d'électrodes comprises dans un stimulateur cardiaque autonome sans sonde.

L'électrode de pointe 23 peut être une électrode de détection ou une électrode de stimulation. Dans une variante, l'électrode 23 est à la fois une électrode de détection et une électrode de stimulation.

Bien que cela ne soit pas visible sur la Figure 1, le corps 31 du stimulateur cardiaque autonome sans sonde 21 peut encapsuler une unité de batterie, un contrôleur et un moyen récepteur, comme un dispositif récepteur RF ou utilise une connexion de communication intracorporelle. Le corps 31 peut également comprendre un moyen émetteur.

Le moyen récepteur du stimulateur cardiaque autonome sans sonde 21 est configuré pour communiquer sans fil avec le moyen émetteur du dispositif implantable sous-cutané 20, en particulier via une connexion de communication intracorporelle.

Le dispositif implantable sous-cutané 21 peut être configuré pour détecter l'activité cardiaque en utilisant l'une des méthodes connues dans l'état de l'art, par exemple au moyen d'un électrocardiogramme (ECG), d'un électrogramme (EGM), d'une cardiographie d'impédance, d'un détecteur acoustique et/ou d'un accéléromètre.

Dans une variante, la communication sans fil entre les dispositifs 20, 21 pourrait également se faire au moyen d'autres méthodes de communication sans fil comme la communication intracorporelle (appelée « intra-body communication » en anglais) ou le couplage inductif.

Dans le système 10, le stimulateur cardiaque autonome sans sonde 21 est ainsi configuré pour recevoir un signal émis par le dispositif implantable sous-cutané 20. Il peut notamment s'agir d'un signal de synchronisation contenant des informations temporelles relatives à une dépolarisation atriale. Un tel signal de synchronisation peut permettre de synchroniser les contractions du ventricule. Le signal de synchronisation peut être utilisé pour la délivrance d'une stimulation dans le ventricule droit en fonction de l'activité cardiaque physiologique du patient qui est détectée par le dispositif implantable sous-cutané 20.

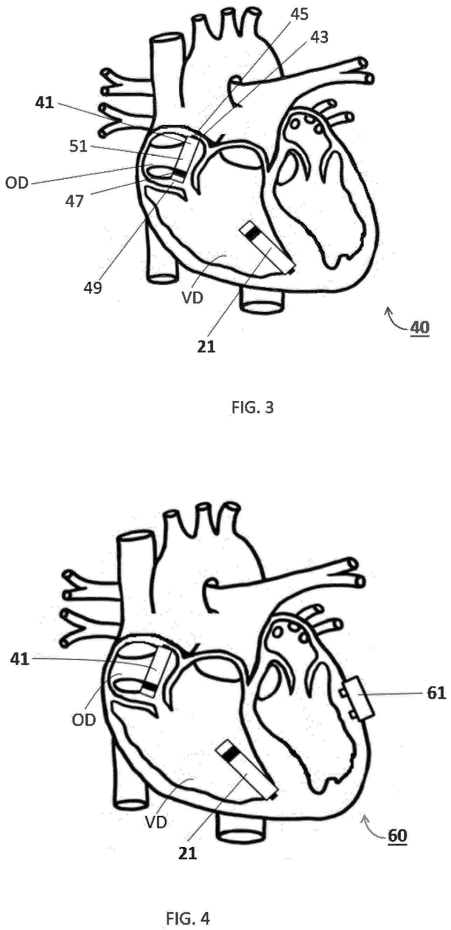

La Figure 3 illustre un système multi-dispositifs 40 selon un deuxième mode de réalisation de la présente invention comprenant deux dispositifs implantables 21, 41.

Les éléments avec les mêmes références numériques déjà utilisées pour la description de la Figure 1 ne seront pas décrits à nouveau en détail, et référence est faite à leurs descriptions ci-dessus.

Le dispositif implantable 21 correspond au stimulateur cardiaque autonome sans sonde 21 déjà décrit en référence à la Figure 1 et à laquelle référence est faite.

Dans le deuxième mode de réalisation, un second stimulateur cardiaque autonome sans sonde 41 est utilisé à la place du dispositif médical implantable sous-cutanée 20 du premier mode de réalisation.

Tel qu'illustré par la Figure 3, le stimulateur cardiaque autonome sans sonde 41 est configuré pour être implanté dans l'oreillette droite (OD).

Le stimulateur cardiaque autonome sans sonde 41 est configuré pour détecter l'activité auriculaire en utilisant l'une des méthodes connues dans l'état de l'art, par exemple au moyen d'un électrocardiogramme (ECG), d'un électrogramme (EGM), d'une cardiographie d'impédance, d'un détecteur acoustique et/ou d'un accéléromètre.

Le stimulateur cardiaque autonome sans sonde 41 est configuré pour analyser des données représentatives d'un ECG. L'analyse des données représentatives d'un ECG rend possible la détection d'au moins une des cinq ondes P, Q, R, S, T du complexe PQRST tel que connu dans l'état de l'art. Un exemple d'un complexe PQRST est illustré à la **Figure 2****.** Le stimulateur cardiaque autonome sans sonde 41 est notamment configuré pour identifier un marqueur temporel correspondant à la détection d'une onde électrique du complexe PQRST.

De même que pour le stimulateur cardiaque autonome sans sonde 21, le stimulateur cardiaque autonome sans sonde 41 comprend une électrode de pointe 43 disposée à une extrémité distale 45 du stimulateur 41, et une électrode annulaire 47 disposée vers une extrémité proximale 49 du stimulateur 41. Les électrodes 43, 47 peuvent former un dipôle récepteur ou un dipôle émetteur. Notons que la présente invention ne se limite pas à l'utilisation d'une électrode de pointe et d'une électrode de type annulaire mais peut être mise en oeuvre au moyen de tous types d'électrodes comprises dans un stimulateur cardiaque autonome sans sonde.

L'électrode de pointe 43 peut être une électrode de détection ou une électrode de stimulation. Dans une variante, l'électrode 43 est à la fois une électrode de détection et une électrode de stimulation.

Bien que cela ne soit pas visible sur la Figure 3, le corps 51 du stimulateur cardiaque autonome sans sonde 41 peut encapsuler une unité de batterie, un processeur, un contrôleur et un moyen émetteur, comme un dispositif émetteur RF. Le corps 51 peut également comprendre un moyen récepteur.

Le moyen émetteur du stimulateur cardiaque autonome sans sonde 41 est configuré pour communiquer sans fil avec le moyen récepteur du stimulateur cardiaque autonome sans sonde 21, en particulier via une connexion de communication intracorporelle.

Dans une variante, la communication sans fil entre les dispositifs 21, 41 pourrait également se faire au moyen d'autres méthodes de communication sans fil comme la communication intracorporelle (appelée « intra-body communication » en anglais) ou le couplage inductif.

Dans une variante qui n'est pas illustrée, un système selon la présente invention comprend un dispositif implantable sous-cutané 20 et deux stimulateurs cardiaques sans sonde 21, 41.

La Figure 4 illustre un système multi-dispositifs 60 selon un troisième mode de réalisation de la présente invention comprenant trois dispositifs implantables 21, 41, 61.

Les éléments avec les mêmes références numériques déjà utilisées pour la description des Figures 1 et 3 ne seront pas décrits à nouveau en détail, et référence est faite à leurs descriptions ci-dessus.

En comparaison avec la deuxième mode de réalisation, le système multi-dispositifs 60 du troisième mode de réalisation comprend un troisième dispositif médical implantable 61. Le système multi-dispositifs 60 est du type système de resynchronisation dit triple chambre, aussi appelé « CRT-P » en anglais pour « Cardiac Resynchronization Therapy-Pacemaker).

Les dispositifs implantables 21, 41 correspondent respectivement aux stimulateurs cardiaques autonomes sans sonde 21, 41 déjà décrits en référence aux Figures 1 et 3 et auxquelles référence est faite.

Le troisième dispositif 61 est un stimulateur cardiaque autonome sans sonde 61 implanté de manière épicardique sur la paroi du myocarde.

Dans chacun des modes de réalisation de la présente invention décrits en référence aux Figures 1, 3 et 4, les dispositifs implantables 20, 21, 41, 61 peuvent contenir aussi bien des moyens récepteurs que des moyens émetteurs, notamment adaptés pour une communication RF. Ainsi, chacun des dispositifs implantables 20, 21, 41, 61 est à la fois configuré pour envoyer un signal et pour recevoir un signal afin de permettre une communication sans fil dans chacun des systèmes 10, 40, 60.

De plus, chacun des dispositifs implantables 20, 21 et 41 sont aptes à déterminer la durée d'un cycle cardiaque.

Le procédé de communication sans fil selon la présente invention se rapporte à la synchronisation d'au moins deux dispositifs implantables tels que 20, 21, 41, 61 compris dans un système comme les systèmes 10, 40 ou 60. Le procédé de communication sans fil selon la présente invention est décrit dans ce qui suit. Les systèmes 10, 40, 60 sont chacun configurés pour mettre en oeuvre ledit procédé de communication. En particulier, le procédé de communication est décrit dans ce qui suit pour un système comprenant un premier dispositif et un deuxième dispositif.

La Figure 5 illustre schématiquement le fonctionnement du procédé de communication selon un premier mode de réalisation de la présente invention.

La Figure 5 représente trois axes temporels 102, 104, 106. L'unité des axes 102, 104, 106 est exprimée en milliseconde (ms).

Un ECG est représenté sur l'axe temporel 102. L'ECG illustré sur l'axe temporel 102 comprend une pluralité de complexe PQRST pour chacun des cycles cardiaques illustrés. La durée d'un cycle cardiaque complet peut être déterminée comme la durée entre deux ondes identiques successives. Sur la Figure 5, la durée du cycle cardiaque est représentée par l'intervalle PP, illustré entre les deux maxima locaux représentant l'onde P du cycle cardiaque. Quatre cycles cardiaques successifs sont annotés sur la Figure 5 de n=1 à n=4.

L'intervalle PP est représentatif de la durée d'un cycle cardiaque complet, et dure typiquement environ une seconde ou plus.

Un premier dispositif est configuré pour analyser l'ECG 102 et pour déterminer une caractéristique de l'ECG afin d'identifier une onde du complexe PQRS, en particulier l'onde P. La caractéristique peut par exemple être le maximum local attribué à l'onde P. Le premier dispositif peut aussi être configuré pour enregistrer l'ECG 102. Le premier dispositif est configuré pour déterminer la durée d'un cycle cardiaque. Ce premier dispositif peut être le dispositif implantable sous-cutané 20, un enregistreur ou le stimulateur cardiaque autonome sans sonde 41 configuré pour être implanté dans l'oreillette droite (OD). Le premier dispositif de la Figure 5 comprend au moins un moyen émetteur, par exemple au moyen une connexion de communication intracorporelle.

Sur l'axe temporel 104 sont représentées les détections de l'onde P pour chaque cycle PP (voir « détection P₁ » à « détection P₄ ») identifiées par le premier dispositif.

L'axe temporel 106 se rapporte à un deuxième dispositif d'un système multi-dispositifs, tel que décrit en référence aux Figures 1, 3 et 4. Ce deuxième dispositif peut être le stimulateur cardiaque autonome sans sonde 21 configuré pour être implanté dans le ventricule droit (VD). Le deuxième dispositif peut être configuré pour déterminer la durée d'un cycle cardiaque. Le deuxième dispositif peut ainsi être apte à délivrer une stimulation ventriculaire.

Le deuxième dispositif de la Figure 5 est indépendant du premier dispositif médical implantable et comprend au moins un moyen récepteur.

Le procédé de la présente invention est un procédé de communication sans fil entre ledit premier dispositif et ledit deuxième dispositif constituant un système multi-dispositif afin de permettre la synchronisation des dispositifs entre eux. Ledit procédé est décrit dans ce qui suit.

Premièrement, considérons le premier cycle cardiaque n=1 illustré sur la Figure 5, pendant lequel le premier et le deuxième dispositif ne sont pas encore synchronisés.

Tel qu'expliqué ci-dessus, le premier dispositif est configuré pour analyser l'ECG et détecter une onde P. Pendant le premier cycle cardiaque n=1, une onde P est détectée par le premier dispositif et est annotée « détection P₁ » sur l'axe temporel 104.

Suite à la détection et l'identification de l'onde P₁, le moyen émetteur du premier dispositif est configuré pour envoyer un signal de synchronisation vers le deuxième dispositif. Le marqueur temporel P_{réf1 n=1} sur l'axe temporel 104 correspond au marqueur temporel qui marque l'envoi du signal de synchronisation pour le premier cycle cardiaque illustré à la Figure 5.

Ce signal de synchronisation est reçu par le moyen récepteur du deuxième dispositif et est indiqué par le marqueur temporel P_{réf2 n=1} sur l'axe temporel 106.

Ensuite, pendant le cycle cardiaque suivant n=2, une onde P est à nouveau détectée et identifiée par le premier dispositif et est annotée « détection P₂ » sur l'axe temporel 104.

Suite à la détection et l'identification de l'onde P₂, le moyen émetteur du premier dispositif est configuré pour envoyer un signal de synchronisation vers le deuxième dispositif. Le marqueur temporel P_{réf1 n=2} sur l'axe temporel 104 correspond au marqueur temporel qui marque l'envoi du signal de synchronisation pour le deuxième cycle cardiaque n=2 illustré à la Figure 5.

Ce signal de synchronisation est reçu par le moyen récepteur du deuxième dispositif et est indiqué par le marqueur temporel P_{réf2 n=2} sur l'axe temporel 106.

Selon la présente invention, la durée du premier cycle cardiaque n=1 est déterminée entre P_{réf1 n=1} et P_{réf1 n=2} (voir « intervalle P_{réf1 n=1} P_{réf1 n=2} » sur la Figure 5). Cela permet de se référer à une référence qui est commune au premier et au deuxième dispositifs.

Dans une variante, la durée entre « détection P₁ » et « détection P₂ » peut permettre de déterminer la durée du premier cycle cardiaque n=1 (voir « intervalle P₁P₂ » sur la Figure 5).

Ensuite, pendant le cycle cardiaque suivant n=3, une onde P est à nouveau détectée et identifiée par le premier dispositif et est annotée « détection P₃ » sur l'axe temporel 104.

Suite à la détection et l'identification de l'onde P₃, le moyen émetteur du premier dispositif est configuré pour envoyer un signal de synchronisation vers le deuxième dispositif. Le marqueur temporel P_{réf1 n=3} sur l'axe temporel 104 correspond au marqueur temporel qui marque l'envoi du signal de synchronisation pour le troisième cycle cardiaque illustré à la Figure 5.

Ce signal de synchronisation est reçu par le moyen récepteur du deuxième dispositif et est indiqué par le marqueur temporel P_{réf2 n=3} sur l'axe temporel 106.

Selon la présente invention, et par analogie avec le premier cycle, la durée du deuxième cycle cardiaque n=2 est déterminée entre P_{réf1 n=2} et P_{réf1 n=3}.

Dans une variante, la durée entre « détection P₂ » et « détection P₃ » peut permettre de déterminer la durée du deuxième cycle cardiaque n+2.

A partir du marqueur temporel P_{réf2 n=2} et de la durée du deuxième cycle cardiaque, un intervalle de synchronisation 108 pour le deuxième dispositif peut être déterminé pour le troisième cycle cardiaque n=3. Cet intervalle de synchronisation 108 correspond à une fenêtre d'écoute, c'est-à-dire que le moyen récepteur du deuxième dispositif est activé sur l'intervalle de synchronisation 108 dans le but de réceptionner un signal de synchronisation émis par le premier dispositif. Un signal ou un message peut ainsi être reçu par le moyen récepteur du deuxième dispositif pendant l'intervalle de synchronisation 108.

La durée de l'intervalle de synchronisation 108 est déterminée de manière à être plus courte que la durée du deuxième cycle cardiaque. L'intervalle de synchronisation 108 peut être de durée inférieure à 0,8s, en particulier, inférieure à 0,4 seconde.

Le début de l'intervalle de synchronisation 108, indiquée par la référence D_{réf2 n=3} est déterminée en fonction du marqueur temporel P_{réf2 n=2} du cycle cardiaque précédent n+2.

L'intervalle de synchronisation 108 étant fonction du marqueur temporel P_{réf2 n=2} du cycle cardiaque précédent n+2, cela permet d'agencer l'intervalle de synchronisation 108 sur le troisième cycle de manière à ce que la réception du signal de synchronisation ait lieu pendant ledit intervalle de synchronisation 108.

La réception du signal de synchronisation est indiquée par le marqueur temporel P_{réf2 n=3}.

La détermination du début D_{réf2 n=3} et de la durée de l'intervalle de synchronisation 108 sur le troisième cycle cardiaque prend notamment en considération l'accélération maximale d'un battement à un autre, qui ne dépasse généralement pas 10 à 40%, en particulier 25 à 35 % de la durée d'un cycle cardiaque.

De plus, la détermination de l'intervalle de synchronisation 108 prend également en compte le temps que met le myocarde pour répondre à une activité auriculaire. Il s'agit d'un paramètre programmable par un médecin afin d'améliorer la réponse physiologique du coeur. Ce paramètre peut correspondre à 0-50%, en particulier 0-30% de la durée d'un cycle cardiaque.

Selon la présente invention, le moyen récepteur du deuxième dispositif est activé pendant l'intervalle de synchronisation 108 et est désactivé en dehors de l'intervalle de synchronisation 108 par le contrôleur du deuxième dispositif.

Ainsi, le moyen récepteur du deuxième dispositif n'est pas continuellement activé pendant la durée d'un cycle cardiaque complet, ce qui permet de réduire la consommation électrique du deuxième dispositif. En effet, tant que le moyen récepteur du deuxième dispositif est activé, il consomme de l'énergie. En activant le moyen récepteur pendant l'intervalle de synchronisation 108 et non plus sur toute la durée d'un cycle cardiaque complet, la consommation énergétique requise pour la communication sans fil aux fins de resynchronisation peut être réduite car la durée de mise sous tension du moyen récepteur est limitée à la durée de l'intervalle de synchronisation 108.

Il ne s'agit toutefois pas d'éteindre l'ensemble des fonctionnalités du deuxième dispositif toutes en même temps, qui met en oeuvre d'autres fonctions comme la détection, le chronométrage ou la stimulation en parallèle de la fonctionnalité du moyen récepteur.

Un intervalle de synchronisation 110 pour le premier dispositif peut également être déterminé à partir du marqueur temporel P_{réf1 n=2} et de la durée du deuxième cycle cardiaque Le début de l'intervalle de synchronisation 110 pour le troisième cycle cardiaque n+3 est indiquée par la référence D_{réf1 n=3}. Le début de l'intervalle de synchronisation 110 D_{réf1 n=3} est donc notamment déterminée en fonction du marqueur temporel P_{réf1 n=2}.

L'intervalle de synchronisation 110 pour le premier dispositif correspond à une fenêtre d'envoi, c'est-à-dire un intervalle pendant lequel le signal de synchronisation est susceptible d'être envoyée, selon l'identification d'une onde P. Pour le troisième cycle cardiaque n+3, l'envoi du signal de synchronisation est indiqué par le marqueur temporel P_{réf1 n=3}.

La détermination des intervalles de synchronisation 108, 110 pour le quatrième cycle cardiaque n+4 et les cycles cardiaques suivants (qui ne sont pas représentés sur la Figure 5) est réalisée de la même manière qu'expliqué ci-dessus pour le troisième cycle cardiaque.

Un avantage du présent procédé est que les intervalles de communication 108, 110 sont ajustés dans le temps en fonction d'un événement cardiaque (la détection d'une onde P) et sont donc adaptés aux caractéristiques physiologiques d'un patient.

Dans une alternative qui n'est pas représentée, l'intervalle de synchronisation 110 peut être agencer sur le troisième cycle n+3 en fonction du marqueur temporel P_{réf1 n=2} et de la durée du deuxième cycle cardiaque de manière à ce que la détection de l'onde P ne soit pas comprise dans l'intervalle de synchronisation 110. Dans ce cas, l'intervalle de synchronisation 110 est décalé vers la droite de l'axe temporel 104 par rapport au premier mode de réalisation. Dans cette alternative, l'intervalle de synchronisation 108 est ainsi également décalé vers la droite de l'axe temporel 104 par rapport au premier mode de réalisation. Cette alternative est particulièrement adaptée pour envoyer un message de synchronisation contenant un ensemble de données.

Notons que le premier dispositif peut envoyer un « signal » ou un « message » de synchronisation. Dans la présente description, un « signal » de synchronisation se réfère à un signal comprenant une information codée sur un seul bit alors qu'un « message » de synchronisation se réfère à un signal comprenant une information codée sur plusieurs bits.

La différence entre un « signal » et un « message » de synchronisation est ainsi relative à la manière dont l'information temporelle de la détection de l'onde P est encodée.

Dans un message de synchronisation, le « timing » de la détection de l'onde P est encodée et permet ainsi l'envoi d'informations davantage détaillées au deuxième dispositif.

Dans un signal de synchronisation, l'information temporelle de la détection de l'onde P est implicite dans le « timing » d'envoi dudit signal. Ainsi, un signal comprenant une information codée sur un seul bit peut être envoyé.

Les marqueurs temporels P_{réf1} et P_{réf2} pour chaque cycle n servent de références temporelles pour synchroniser les intervalles de synchronisation 108, 110 entre eux. Les marqueurs temporels P_{réf1} et P_{réf2} sont notamment utilisés pour s'assurer que les intervalles de synchronisation 108, 110 se chevauchent, voire s'alignent.

Ainsi, le marqueur temporel P_{réf2} est utilisé pour ajuster l'activation du moyen récepteur du deuxième dispositif.

La Figure 6 illustre schématiquement le fonctionnement du procédé de communication selon un deuxième mode de réalisation de la présente invention.

De même qu'à la Figure 5, la Figure 6 représente trois axes temporels 202, 204, 206. L'unité des axes 202, 204, 206 est exprimée en milliseconde (ms).

Un ECG est représenté sur l'axe temporel 202. L'ECG comprend un complexe PQRST. La durée d'un cycle cardiaque complet peut être déterminée comme la durée entre eux ondes identiques successives.

Sur l'axe temporel 204 est représenté un intervalle de synchronisation 210 d'un premier dispositif d'un système multi-dispositifs, tel que décrit en référence aux Figures 1, 3 et 4.

Sur l'axe temporel 206 est représenté un intervalle de synchronisation 208 d'un deuxième dispositif d'un système multi-dispositifs, tel que décrit en référence aux Figures 1, 3 et 4. Ce deuxième dispositif peut être le stimulateur cardiaque autonome sans sonde 21 configuré pour être implanté dans le ventricule droit (VD). Le deuxième dispositif peut ainsi être apte à délivrer une stimulation ventriculaire.

Le deuxième dispositif de la Figure 6 est indépendant du premier dispositif médical implantable et comprend au moins un moyen récepteur.

Pour un cycle cardiaque n, l'intervalle de synchronisation 208 du deuxième dispositif est déterminé de la même manière que celle décrite pour l'intervalle de synchronisation 108 du deuxième dispositif au troisième cycle du premier mode de réalisation (voir figure 5). Autrement dit, le début D_{réf2 n} de l'intervalle de synchronisation 208 du deuxième dispositif illustré sur la Figure 6 est fonction du deuxième marqueur temporel P_{réf2 n-1} du cycle cardiaque précédent n-1 (qui n'est pas représenté sur la Figure 6).

De même, pour un cycle cardiaque n, l'intervalle de synchronisation 210 du premier dispositif est déterminé de la même manière que celle décrite pour l'intervalle de synchronisation 110 du premier dispositif au troisième cycle du premier mode de réalisation (voir figure 5). Autrement dit, le début D_{réf1 n} de l'intervalle de synchronisation 210 du premier dispositif illustré sur la Figure 6 est fonction du premier marqueur temporel P_{réf1 n-1} du cycle cardiaque précédent n-1 (qui n'est pas représenté sur la Figure 6).

Pendant l'intervalle de synchronisation 208, le moyen émetteur du premier dispositif peut émettre des impulsions de durée T_{bit} espacées de périodes de repos T_{off} tel que représenté sur l'axe temporel 204 de la Figure 6. Pendant les périodes de repos T_{off} le moyen émetteur n'envoie pas d'impulsion. Les durées T_{bit} peuvent être égale à 500 microsecondes (µs) et « T_{off} » peuvent être égale à 9,5 millisecondes (ms).

Dès la détection d'une onde P, indiquée par « détection P » sur la figure 6, le moyen émetteur du premier dispositif utilise le premier créneau disponible défini par T_{bit} pour émettre un signal de synchronisation. Ainsi, bien que la Figure 6 représente une pluralité de créneaux T_{bit}, un seul créneau est utilisé par le moyen émetteur pour envoyer le signal de synchronisation.

Autrement dit, lorsque le premier dispositif détecte une onde P, l'émetteur du premier dispositif est configuré pour envoyer un signal de synchronisation au premier bloc T_{bit} qui suit la détection de l'onde P, qui est indiqué par le bloc T_{bit P} sur la Figure 6. L'envoi du signal de synchronisation est marqué par le marqueur temporel P_{réf1 n}. Le marqueur temporel P_{réf1 n} peut indiquer le début de l'envoi du signal ou la fin de l'envoi du signal.

Afin de réduire davantage la consommation énergétique du deuxième dispositif, dans le deuxième mode de communication, le moyen récepteur du deuxième dispositif est activé puis désactivé par le contrôleur par intervalle durant l'intervalle de synchronisation 208.

L'intervalle de synchronisation 208 du deuxième dispositif comprend ainsi une pluralité de créneaux temporels d'activation « mₒₙ » et une pluralité de créneaux temporels de désactivation « m_{off} ». Autrement dit, l'intervalle de synchronisation 208 comprend une succession de blocs « m », eux-mêmes comprenant chacun un créneau mₒₙ et un créneau m_{off}, tel qu'illustré à la Figure 6 sur l'axe temporel 206.

Pendant un créneau temporel d'activation « mₒₙ », le moyen récepteur est activé, il est configuré pour recevoir un signal, notamment le signal de synchronisation émis par le premier dispositif.

Pendant un créneau temporel désactivation « m_{off} », le moyen récepteur est désactivé, c'est-à-dire qu'il est éteint et ne consomme pas d'énergie.

Dans l'exemple de la Figure 6, chaque créneau temporel d'activation « mₒₙ » est de même durée.

Dans l'exemple de la Figure 6, chaque créneau temporel désactivation « m_{off} » est de même durée.

La durée de chaque créneau mₒₙ peut être égale ou plus courte que la durée de chaque créneau m_{off}.

Les intervalles de synchronisation 208, 210 du cycle cardiaque n sont synchronisés entre eux à partir des marqueurs temporels P_{réf2 n-1} et P_{réf1 n-1} du cycle cardiaque précédent n-1, tel qu'expliqué en référence à la Figure 5.

En outre, la pluralité de créneaux temporels d'activation mₒₙ prédéfinis du deuxième dispositif est synchronisée à la pluralité de créneau T_{bit} du premier dispositif au moyen du deuxième marqueur temporel P_{réf2 n-1} et du premier marqueur temporel P_{réf1 n-1} du cycle cardiaque précédent n-1.

Tel qu'illustré à la Figure 6, le signal de synchronisation est reçu par le récepteur du deuxième dispositif durant le créneau d'activation indiqué par « m_{on P} ». La réception du signal est marquée par le marqueur temporel P_{réf2 n}.

La durée d'un créneau temporel d'activation mₒₙ peut représenter 0,3 à 50 % de la durée d'un créneau temporel de désactivation m_{off}. La durée d'un créneau temporel d'activation mₒₙ dépend de la qualité du récepteur et du temps dont il a besoin pour détecter le signal de synchronisation. En particulier, la durée d'un créneau temporel d'activation mₒₙ peut représenter 5 à 10 % de la durée d'un créneau temporel de désactivation m_{off}.

En considérant un intervalle synchronisation 208 d'environ 350ms et un ratio mₒₙ=10% de m (m=mₒₙ+m_{off}), il s'avère que l'amélioration du cycle d'activation du moyen récepteur du deuxième dispositif à des fins de synchronisation peut réduire la consommation d'énergie de 0,1 à 3,5 %. Par exemple, un bloc m peut être égale à une durée de 10ms, le bloc m comprenant un créneau mₒₙ de 1ms et un créneau m_{off} de 9ms.

Dans une variante avantageuse, le moyen récepteur du deuxième dispositif est désactivé pour le reste du cycle cardiaque donné, une fois que le marqueur temporel P_{réf2 n} a été déterminé, afin d'économiser davantage d'énergie. Dans l'exemple de la figure 6, cela se traduirait par la désactivation du moyen récepteur à partir du bloc m_{on P} jusqu'à la fin du cycle cardiaque n.

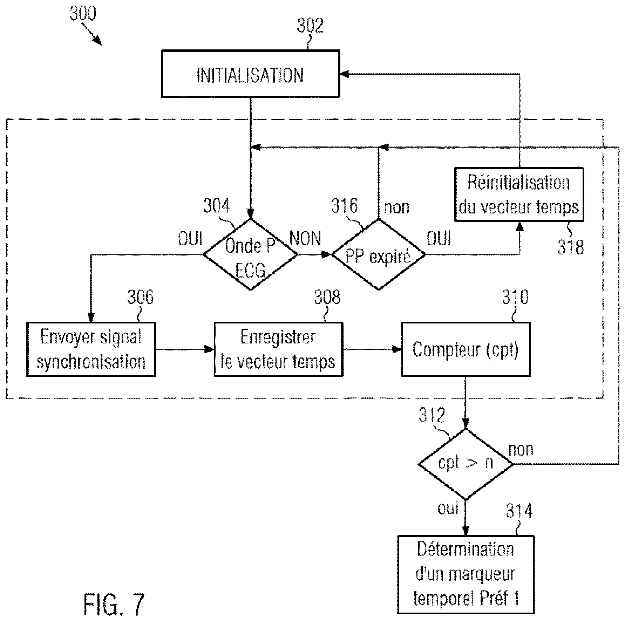

La Figure 7 illustre au moyen d'un organigramme 300 des étapes d'initialisation du procédé mis en oeuvre par le premier dispositif. Ces étapes d'initialisation sont mises en oeuvre de manière asynchrone par rapport au deuxième dispositif. Les étapes du procédé de l'organigramme 300 s'appliquent aux modes de réalisation décrits en référence aux Figures 5 et 6.

Tel qu'illustré par l'organigramme de la Figure 7, l'initialisation 302 comprend la détection d'un marqueur temporel d'un cycle cardiaque à une étape 304. Ce marqueur temporel correspond à la détection d'une onde électrique prédéfinie du complexe PQRST. Tel qu'expliqué précédemment, le premier dispositif est en effet configuré pour analyser les données représentatives d'un ECG.

De préférence, le marqueur temporel correspond à la détection de l'onde P qui est la première onde détectable du complexe PQRST. L'onde P apparaît quand le stimulus (ou l'influx) se propage au myocarde auriculaire, dépolarisant les oreillettes.

Dans ce qui suit, il est ainsi fait référence à la détection de l'onde P, qui a été choisie comme onde électrique prédéfinie dans le mode de réalisation de la Figure 7, tel qu'indiqué à l'étape 304 de l'organigramme 300. Il faut garder à l'esprit que le choix de l'onde P n'est toutefois pas limitatif, et qu'une autre onde du complexe PQRST peut être considérée, notamment l'onde R.

Si une onde P est détectée à l'étape 304, un signal de synchronisation est émis à une étape 306 à destination du deuxième dispositif.

Le signal de synchronisation représente ainsi le « timing » de la détection de l'onde P. Le signal de synchronisation pour chaque cycle cardiaque permet d'indiquer qu'une dépolarisation des oreillettes, en particulier de l'oreillette droite, a été détectée.

A une étape 308, qui suit l'étape 306, un vecteur temps caractérisant le moment (le « timing ») auquel a été émis le signal de synchronisation à l'étape 306 est sauvegardé. Le vecteur temps peut être sauvegardé dans une mémoire de type FIFO pour « First In First Out » en anglais. En d'autres termes, un marqueur temporel marquant l'envoi du signal de synchronisation est déterminé à l'étape 308.

Ensuite, à une étape 310, le vecteur temps est incrémenté dans un compteur de manière à ce que lorsqu'une pluralité « n » de marqueurs temporels pour n cycles cardiaques - et donc d'autant de vecteur temps - ont été incrémentés à une étape 312, ils peuvent être comparés entre eux afin de définir un marqueur temporel dit de référence P_{réf1} sur la base des n marqueurs temporels.

Ainsi, à une étape 314, la détermination d'un marqueur temporel de référence P_{réf1} marquant l'envoi d'un signal de synchronisation est effectuée en prenant en considération le « timing » de la détection de l'onde P pour un nombre n de cycles cardiaques (par exemple en calculant une moyenne sur n cycles cardiaques), en particulier n étant supérieur ou égal à trois, à condition que les n cycles cardiaques soient considérés comme des cycles cardiaques réguliers. Des cycles cardiaques sont considérés réguliers quand l'accélération maximale cycle à cycle ne dépasse pas 25%. Par exemple, avec un cycle cardiaque moyen (qui peut être défini par un intervalle PP) de 1 s, un intervalle PP de durée supérieure ou égale à 750ms est considéré stable.

Si, à l'étape 304, aucune onde P n'est détectée, il est vérifié à une étape 316 que la durée du cycle cardiaque n'a pas encore expiré. La durée d'un cycle cardiaque peut être déterminée par la durée entre deux marqueurs temporels successifs marquant l'envoi du signal ou deux ondes P successives par exemple. Notons qu'en phase d'initialisation, un intervalle PP dit « d'initialisation » prédéterminé peut être utilisé.

Selon la présente invention, la durée d'un cycle cardiaque peut aussi être déterminée au moyen du premier dispositif et/ou du deuxième dispositif via un moyen de détection, qui permet par exemple d'obtenir le tracé d'un ECG.

Si la durée du cycle cardiaque n'est pas considérée comme ayant expirée à l'étape 316, alors le premier dispositif continue d'analyser l'ECG à l'étape 304.

Au contraire, si la durée du cycle cardiaque est considérée comme ayant expirée à l'étape 316, le vecteur temps est réinitialisé à une étape 318 et revient ainsi à la première étape 302 d'initialisation.

Suites aux étapes d'initialisation du procédé, un marqueur temporel de référence P_{réf1} indicateur du « timing » de l'envoi d'un signal de synchronisation suite à la détection d'une onde P est ainsi déterminé à l'étape 314 de l'organigramme 300.

Ce marqueur temporel de référence P_{réf1} est par exemple représenté aux Figures 5 et 6.

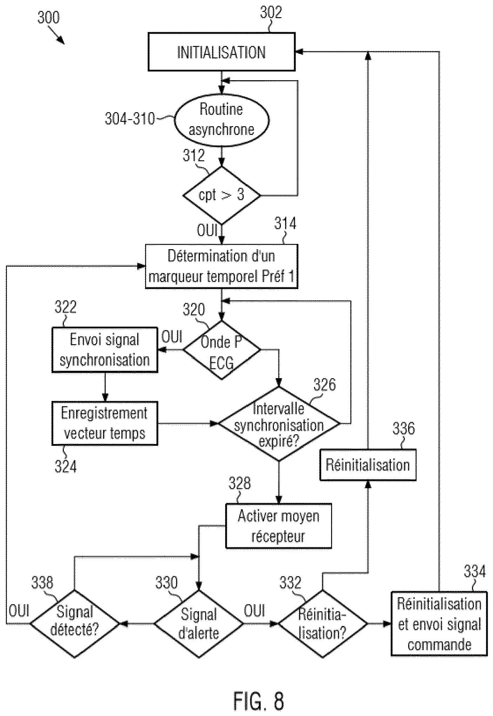

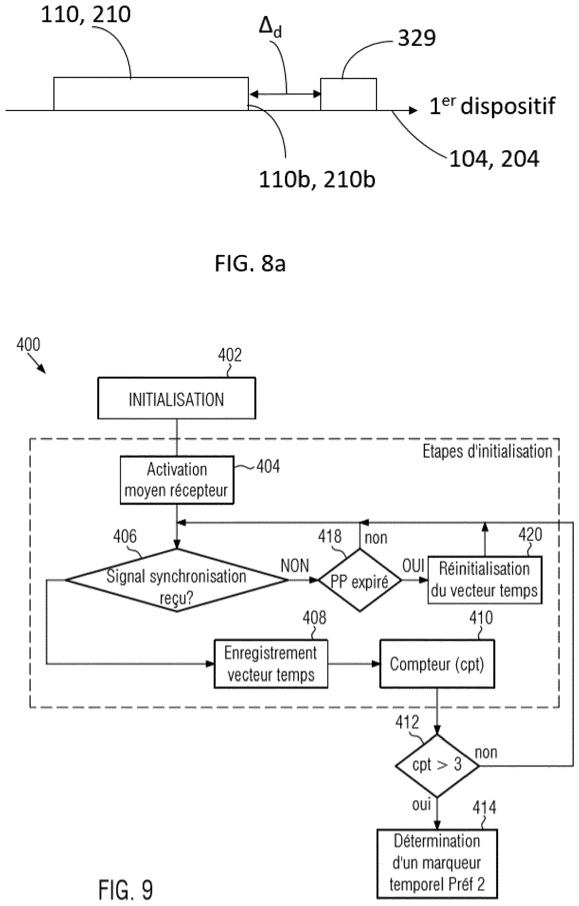

La Figure 8 illustre la suite de l'organigramme 300 de la Figure 7, c'est-à-dire des étapes opérationnelles du procédé qui suivent l'étape 314 à laquelle un marqueur temporel référence P_{réf1} est déterminé. Ainsi, les étapes du procédé décrites au moyen de la Figure 8 s'appliquent aux modes de réalisation décrits en référence aux Figures 5 et 6.

Lors des étapes opérationnelles du procédé un intervalle de synchronisation pour le premier dispositif est utilisé. Un tel intervalle de synchronisation 110, 210 a déjà été décrit en relation avec les Figures 5 et 6 auxquelles références est faite.

Si une onde P pour le cycle cardiaque suivant n+1 est détectée à une étape 320 alors un signal de synchronisation correspondant est envoyé à une étape 322 à destination du deuxième dispositif. Le signal de synchronisation est porteur d'informations physiologiques relative à la détection du complexe PQRST et permet la délivrance d'une thérapie synchronisée.

A une étape 324, qui suit l'émission du signal de synchronisation de l'étape 322, un vecteur temps caractérisant le moment auquel a été émis le signal de synchronisation à l'étape 322 est sauvegardé. Le vecteur temps peut être sauvegardé dans une mémoire de type FIFO pour « First In First Out » en anglais.

Il est ensuite vérifié à une étape 326 si l'intervalle de synchronisation 110, 210 pour le cycle cardiaque n+1 a expiré. Si tel n'est pas le cas, le premier dispositif continue d'être activé et d'attendre la détection une onde P pour le cycle cardiaque n+1 afin de pouvoir envoyer un signal de synchronisation.

Si une onde P est détectée à l'étape 320, un signal de synchronisation est émis à l'étape 322. Puis, s'il est confirmé à l'étape 326 que la durée de l'intervalle de synchronisation 110, 210 a expiré alors le premier dispositif est configuré pour activer un moyen récepteur du premier dispositif à une étape 328.

L'étape 328 est davantage décrit ci-après en référence à la Figure 8a.

Tel qu'illustré à la Figure 8a, le moyen émetteur du premier dispositif est configuré pour être activé pendant un intervalle de synchronisation 110, 210. Le premier dispositif peut en outre comprendre un moyen récepteur configuré pour être activé pendant un intervalle 329 qui est plus court que l'intervalle de synchronisation 110, 210. Le moyen récepteur du premier dispositif peut être activé avec un décalage prédéterminé Δ_{d} à partir de la fin 110a, 210a de l'intervalle de synchronisation 110, 210, tel qu'illustré sur l'axe temporel 104, 204 de la Figures 8a.

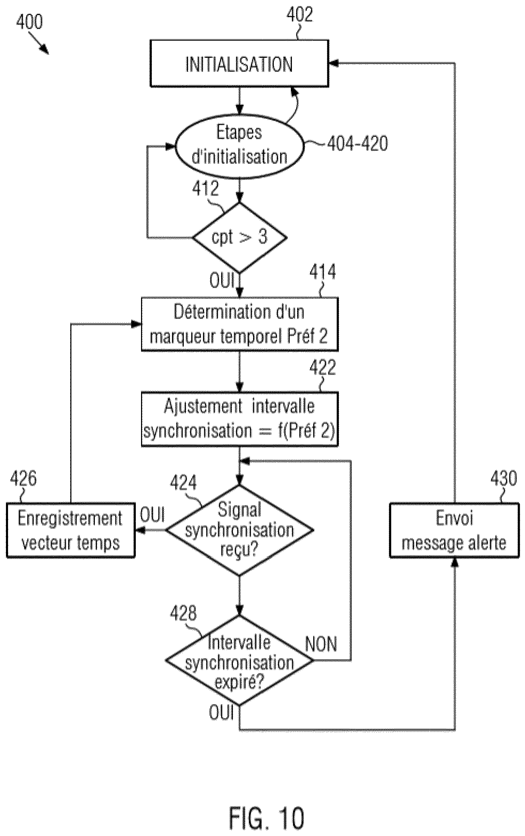

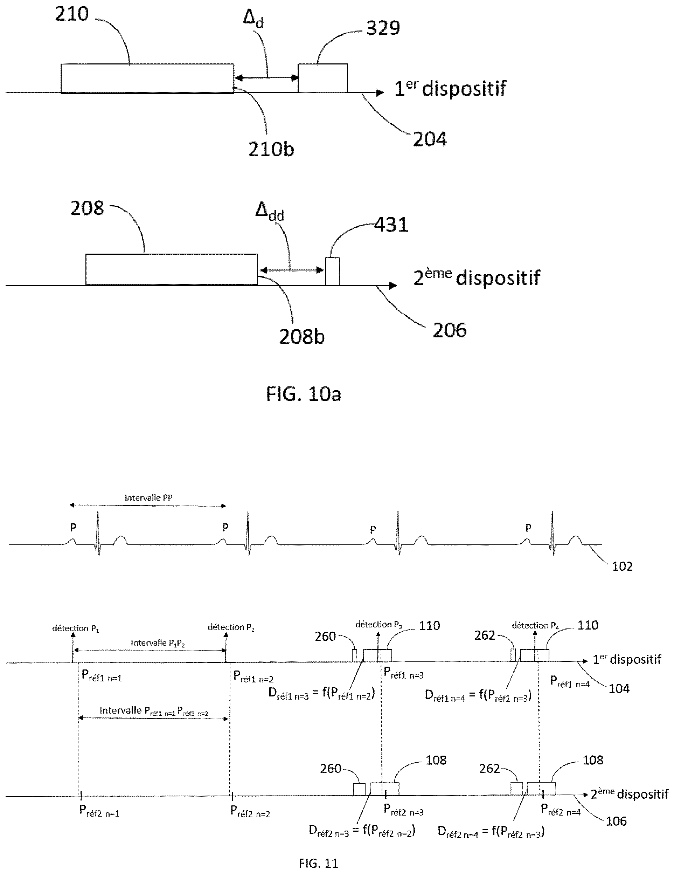

Pendant l'intervalle 329, le moyen récepteur du premier dispositif est activé pour détecter un éventuel signal d'alerte émis par le deuxième dispositif. L'envoi d'un signal d'alerte par le deuxième dispositif est davantage décrit ci-après en référence aux Figures 9 et 10.

Si, à une étape 330, le moyen récepteur du premier dispositif reçoit un signal d'alerte, il est déterminé à une étape 332 s'il faut réinitialiser le procédé et envoyer un signal de commande afin de requérir la délivrance d'une stimulation au niveau de l'oreillette droite (étape 334) ou seulement réinitialiser le procédé sans requérir la délivrance d'une stimulation (étape 336).

Dans le cas où aucun signal d'alerte n'est détecté pendant l'intervalle 329 à une étape 338, le premier dispositif continue de fonctionner d'une manière quasi-synchronisée en utilisant l'intervalle de synchronisation 110, 210 pour la communication sans fil avec le deuxième dispositif.

L'organigramme 300 illustré par les Figures 7 et 8 représente ainsi un algorithme mis en oeuvre par le premier dispositif du système multi-dispositif.

Le système multi-dispositif selon la présente invention comprend au moins un deuxième dispositif, configuré pour être implanté dans le ventricule droit, et dans lequel est aussi implémenté un algorithme. Le deuxième dispositif est indépendant du premier dispositif. Toutefois, le deuxième dispositif met en oeuvre un algorithme complémentaire à celui du premier dispositif. L'algorithme mis en oeuvre par le deuxième dispositif est décrit dans ce qui suit au moyen de d'un organigramme 400 illustré aux Figures 9 et 10.

Les Figures 9 et 10 représente un organigramme 400 du procédé mise en oeuvre par le deuxième dispositif.

L'organigramme 400 de la Figure 9 représente des étapes d'initialisation du procédé mises en oeuvre par le deuxième dispositif. Ces étapes d'initialisation sont mises en oeuvre de manière asynchrone par rapport au premier dispositif. Les étapes du procédé de l'organigramme 400 peuvent s'appliquer aux modes de réalisation décrits en référence aux Figures 5 et 6.

Pendant ces étapes d'initialisation mises en oeuvre par le deuxième dispositif, le moyen récepteur du deuxième dispositif est activé de manière continue car le « timing » de la réception d'un signal de synchronisation n'est pas encore estimé.

Pour commencer, tel qu'illustré par l'organigramme de la Figure 9, l'initialisation 402 comprend l'activation du moyen récepteur du deuxième dispositif à une étape 404.

Il est analysé à une étape 406 si un signal de synchronisation est reçu par le moyen récepteur du deuxième dispositif.

Si un signal de synchronisation est effectivement reçu alors, à une étape 408, un vecteur temps caractérisant le moment (le « timing ») auquel a été reçu le signal de synchronisation à l'étape 406 est sauvegardé. Le vecteur temps peut être sauvegardé dans une mémoire de type FIFO pour « First In First Out » en anglais.

Ensuite, à une étape 410, le vecteur temps est incrémenté dans un compteur de manière à ce que lorsqu'une pluralité n de vecteurs temps pour n cycles cardiaques - et donc d'autant de signaux de synchronisation reçus - ont été incrémentés à une étape 412, ils peuvent être comparés entre eux afin de définir pour le deuxième dispositif un marqueur temporel dit de référence P_{réf2}.

Ainsi, à une étape 414, la détermination d'un marqueur temporel de référence P_{réf2} est effectuée en prenant en considération le « timing » de la réception du signal de synchronisation pour un nombre n de cycles cardiaques, en particulier n étant supérieur ou égal à trois, à condition que les n cycles cardiaques soient considérés comme des cycles cardiaques réguliers. Des cycles cardiaques sont considérés réguliers quand l'accélération maximale cycle à cycle ne dépasse pas 25%. Par exemple, avec un cycle cardiaque moyen (qui peut être défini par un intervalle PP) de 1 s, un intervalle PP de durée supérieure ou égale à 750ms est considéré stable. Le marqueur temporel de référence P_{réf2} peut notamment être déterminé en faisant une moyenne des « timings » de la réception du signal de synchronisation pour un nombre n de cycles cardiaques.

Si, à l'étape 406, aucun signal de synchronisation n'est reçu, il est vérifié à une étape 418 que la durée du cycle cardiaque considéré n'a pas encore expiré. La durée d'un cycle cardiaque peut être déterminée par la durée entre deux ondes P successives par exemple.

Ainsi, le deuxième dispositif peut être configuré pour estimer la durée d'un cycle cardiaque au moyen des informations temporelles contenues dans deux signaux de synchronisation reçus successivement.

Dans une variante, le deuxième dispositif peut comprendre un moyen de détection permettant d'obtenir le tracé d'un ECG par exemple, et d'en déduire la durée d'un cycle cardiaque. Si la durée du cycle cardiaque n'est pas considérée comme ayant expirée à l'étape 418, alors le deuxième dispositif continue d'attendre la réception d'un signal de synchronisation à l'étape 406.

Au contraire, si la durée du cycle cardiaque est considérée comme ayant expirée à l'étape 418, le vecteur temps est réinitialisé à une étape 420 et revient ainsi à l'étape 406.

Suites aux étapes d'initialisation du procédé, un marqueur temporel de référence P_{réf2} indicateur du « timing » de la réception d'un signal de synchronisation et relatif à la détection d'une onde P dans les oreillettes, est ainsi déterminé à l'étape 414 de l'organigramme 400.

Un tel marqueur temporel de référence P_{réf2} est par exemple représenté sur l'axe temporel 106, 206 des Figures 5 et 6.

La Figure 10 illustre la suite de l'organigramme 400 de la Figure 9, c'est-à-dire des étapes opérationnelles du procédé qui suivent l'étape 414 à laquelle le marqueur temporel référence P_{réf2} est déterminé. Selon le deuxième mode de réalisation, pendant les étapes opérationnelles du procédé, en particulier à partir de l'étape 422 décrite ci-après, le moyen récepteur du deuxième dispositif n'est plus activé de manière continue mais par intervalle pendant l'intervalle de synchronisation 208. Référence est faite à la description de la Figure 6 pour l'activation du moyen récepteur par intervalle pendant des blocs mₒₙ de l'intervalle de synchronisation 208.

Tel qu'illustré par la Figure 10, à une étape 422, qui suit l'étape 414, le deuxième dispositif met à jour et ajuste l'intervalle synchronisation 208 par rapport à la valeur du marqueur temporel référence P_{réf2} déterminé à l'étape 414. L'intervalle de synchronisation 208 d'un cycle cardiaque n est ajusté en fonction de la valeur du marqueur temporel référence P_{réf2} déterminée au cycle cardiaque n-1 précédent ou en fonction de la moyenne des valeurs du marqueur temporel référence P_{réf2} déterminée d'une pluralité de cycle cardiaque précédent.

A une étape 424, le moyen récepteur est activé sur l'intervalle synchronisation 208 pendant une pluralité de créneaux temporels d'activation mₒₙ, tel qu'illustré à la Figure 6.

Il est analysé à l'étape 424 si un signal de synchronisation émis par le premier dispositif, est reçu par le moyen récepteur du deuxième dispositif.

Si un signal de synchronisation est effectivement reçu alors, à une étape 426, un vecteur temps caractérisant le moment (le « timing ») auquel a été reçu le signal de synchronisation est sauvegardé. Le vecteur temps peut être sauvegardé dans une mémoire de type FIFO pour « First In First Out » en anglais.

Le vecteur temps est utilisé à l'étape 414 afin de mettre à jour le « timing » de la réception du signal de synchronisation, c'est-à-dire le marqueur temporel de référence P_{réf2} qui sera utilisé pour le prochain cycle cardiaque. Le marqueur temporel de référence P_{réf2} est ainsi actualisé battement après battement en étant calculé sur la base des marqueurs temporels de référence P_{réf2} des précédents battements, par exemple sur la base des deux précédents cycles cardiaques n-2 et n-1 qui ont précédé le cycle cardiaque n considéré.

Si, à l'étape 424, aucun signal de synchronisation n'est reçu, il est vérifié à une étape 428 que la durée de l'intervalle de synchronisation 208 n'a pas encore expiré.

Si la durée de l'intervalle de synchronisation 208 n'a pas encore expiré à l'étape 428, alors le deuxième dispositif continue d'attendre la réception d'un signal de synchronisation à l'étape 424.

Au contraire, si la durée de l'intervalle de synchronisation 208 a expiré à l'étape 428 sans qu'un signal de signalisation n'ait été reçu, le deuxième dispositif est configuré pour envoyer un signal d'alerte à une étape 430. Pour se faire, le deuxième dispositif comprend en outre un moyen émetteur.

A noter que le deuxième dispositif ne connaît pas la cause de l'absence de réception de signal de signalisation. Il peut s'agir d'un échec de communication, ou bien qu'une onde P n'ait pas été détectée par le premier dispositif. Dans les deux cas, le deuxième dispositif envoie un signal d'alerte à l'étape 430.

L'étape 430 est davantage décrit ci-après en référence à la Figure 10a.

A l'étape 430, le deuxième dispositif est configuré pour activer un moyen émetteur. Tel qu'illustré à la Figure 10a, le moyen émetteur du deuxième dispositif est configuré pour être activé pendant un intervalle 431 qui est plus court que l'intervalle de synchronisation 208.

Le signal d'alerte peut être envoyé avec un décalage prédéterminé Δ_{dd} après la fin 208b de l'intervalle de synchronisation 208 tel que représenté sur l'axe temporel 206 de la Figure 10a.

Ainsi, pendant l'intervalle 431, le moyen émetteur du deuxième dispositif est activé pour envoyer au premier dispositif un signal d'alerte. L'intervalle 431 d'activation du moyen émetteur du deuxième dispositif est plus court que l'intervalle 329 d'activation du moyen récepteur du premier dispositif.

L'intervalle 431 d'activation du moyen émetteur du deuxième dispositif peut être utilisé à double escient : à la fois pour informer le premier dispositif de l'échec de la communication (du fait de l'absence de réception d'un signal de synchronisation) - le premier dispositif sachant que son moyen émetteur a effectivement envoyé un signal de synchronisation, ou pour requérir une stimulation artificielle et locale de l'oreillette droite.

Le procédé de communication de la présente invention permet de synchroniser la thérapie délivrée par le système multi-dispositifs et permet d'économiser une quantité considérable d'énergie.

Cependant, les patients porteurs d'un tel système multi-dispositifs peuvent souffrir d'épisodes au cours desquels l'activité auriculaire devient rapide et irrégulière, appelée tachyarythmie supraventriculaire. Dans ce cas, la thérapie délivrée au ventricule doit être indépendante de l'activité auriculaire.

De plus, un patient souffrent d'arythmie auriculaire a besoin de découpler temporairement la thérapie double-chambre (ou triple-chambre) afin de désynchroniser le ventricule (ou les ventricules) de l'oreillette.

La Figure 11 illustre une variante du premier et du deuxième mode de réalisation avantageusement adaptée au cas des troubles du rythme cardiaque susmentionnés.

La Figure 11 est basée sur le diagramme 100 de la Figure 5, auquel est ajouté sur chacun des axes temporels 104, 106 un intervalle dit de démarrage 260, 262 qui précède les intervalles de synchronisation 208, 210 pour chacun du premier dispositif et du deuxième dispositif.

Pendant l'intervalle de démarrage 260, le moyen émetteur du premier dispositif est configuré pour envoyer un signal d'autorisation de démarrage au moyen récepteur du deuxième dispositif.

Ainsi, pendant l'intervalle de démarrage 262, le moyen receveur du deuxième dispositif est configuré pour recevoir le signal d'autorisation de démarrage émis par le deuxième dispositif.

Les intervalles de démarrage 260, 262 permet d'autoriser ou non la communication entre le premier et le deuxième dispositif.

Il est également possible que le premier dispositif attende un signal d'accusé de réception qui est envoyé par un moyen émetteur du deuxième dispositif afin de s'assurer que l'information a été correctement reçue par le deuxième dispositif.

Cela permet d'éviter que de l'énergie soit inutilement dissipée pour activer le moyen récepteur du deuxième dispositif, par exemple pendant des épisodes d'arythmie cardiaque.

Les intervalles de démarrage 260, 262 peuvent également fournir plus simplement une fonction « d'interrupteur », afin de pouvoir passer d'un mode de fonctionnement mono-chambre (c'est-à-dire un mode où les dispositifs ne sont pas synchronisés) à un mode de fonctionnement double-chambre (ce qui nécessite la synchronisation des dispositifs).

Le procédé de communication sans fil et le système multi-dispositifs configuré pour mettre en oeuvre ledit procédé de la présente invention permettent d'ajuster et d'adapter des fenêtres de communication - qui correspondent aux intervalles de synchronisation - au rythme électrophysiologique du patient. En effet, chaque dispositif du système est capable de déterminer un marqueur temporel associé à la détection des ondes P (ou tout autre onde du complexe PRQST) et de réarranger la fenêtre de communication en fonction du marqueur temporel ainsi déterminé pour chaque cycle cardiaque.

## Revendications

1. Procédé de communication dans un système comprenant plusieurs dispositifs médicaux implantables,
dans lequel un premier dispositif comprend au moins un moyen de détection d'un signal représentatif de l'activité auriculaire, un moyen émetteur, et un contrôleur configuré pour analyser un signal représentatif de l'activité auriculaire, et
un deuxième dispositif, indépendant du premier dispositif médical implantable, comprenant au moins un moyen récepteur et un contrôleur,
le procédé comprenant:
A) une étape de synchronisation temporelle du premier dispositif avec le second dispositif,
par envoi d'un signal de synchronisation du moyen émetteur du premier dispositif vers le deuxième dispositif,
le signal de synchronisation étant envoyé suite à l'identification, au moyen du contrôleur du premier dispositif, d'une onde électrique prédéfinie du complexe PQRS d'un signal représentatif de l'activité auriculaire,
B) une étape de détermination de la durée d'un cycle cardiaque,
les étapes A et B étant suivies :
C) d'une étape de détermination d'un intervalle de synchronisation (108, 208) dont la durée de l'intervalle de synchronisation est déterminée en fonction de la durée du cycle cardiaque de sorte à être plus courte que la durée du cycle cardiaque, et dont le début de l'intervalle de synchronisation (108, 208) est déterminée en fonction du signal de synchronisation,
D) d'une étape d'activation du moyen récepteur du deuxième dispositif pendant l'intervalle de synchronisation (108, 208),
le moyen récepteur du deuxième dispositif étant désactivé en dehors de l'intervalle de synchronisation (108, 208) par le contrôleur du deuxième dispositif.

2. Le procédé selon la revendication 1, dans lequel pour un cycle cardiaque, un premier marqueur temporel (P_{réf 1}) marquant l'envoi du signal de synchronisation par le moyen émetteur du premier dispositif est déterminé, et
un deuxième marqueur temporel (P_{réf 2}) marquant la réception du signal de synchronisation par le moyen récepteur du deuxième dispositif est déterminé, et l'intervalle de synchronisation (110, 210) du premier dispositif pour un cycle cardiaque suivant est déterminé en fonction du premier marqueur temporel (P_{réf 1}), l'intervalle de synchronisation (108, 208) du deuxième dispositif pour un cycle cardiaque suivant est déterminé en fonction du deuxième marqueur temporel (P_{réf 2}).

3. Le procédé selon la revendication 2, dans lequel pendant l'intervalle de synchronisation (108, 208) du deuxième dispositif :
le moyen récepteur du deuxième dispositif est activé pendant une pluralité de créneaux temporels d'activation prédéfinis (mₒₙ) et est désactivé pendant une pluralité de créneaux temporels de désactivation prédéfinis (m_{off}),
la pluralité de créneaux temporels d'activation prédéfinis (mₒₙ) du deuxième dispositif étant répartie sur l'intervalle de synchronisation (108, 208) du deuxième dispositif de manière à être synchronisée avec des créneaux d'impulsion de signal du premier dispositif en fonction du premier marqueur temporels (P_{réf 1}) et du deuxième marqueur temporel (P_{réf 2}).

4. Le procédé selon la revendication 3, dans lequel le moyen émetteur du premier dispositif est configuré pour envoyer un seul signal de synchronisation par cycle cardiaque.

5. Le procédé selon la revendication 3 ou 4, dans lequel :
- chacun des créneaux temporels d'activation prédéfinis (mₒₙ) est de même durée et
- chacun des créneaux temporels de désactivation prédéfinis (m_{off}) est de même durée et
- chacun des créneaux temporels d'activation (mₒₙ) et de désactivation (m_{off}) prédéfinis se succèdent alternativement dans l'intervalle de synchronisation (108, 208).

6. Le procédé selon la revendication 5, dans lequel la durée d'un créneau temporel d'activation (mₒₙ) est plus courte ou égale que la durée d'un créneau temporel de désactivation (m_{off}).

7. Le procédé selon la revendication 5 ou 6, dans lequel la durée d'un créneau temporel d'activation (mₒₙ) représente entre 0,3 à 50% de la durée d'un créneau temporel de désactivation (m_{off}), en particulier 5 à 10%.

8. Le procédé selon la revendication 3, dans lequel la durée d'une impulsion pendant laquelle le moyen émetteur du premier dispositif est configuré pour émettre un signal de synchronisation correspond à 25 à 80 % de la durée d'un créneau temporel d'activation (mₒₙ), en particulier à 50%.

9. Le procédé selon la revendication 2, dans lequel le premier marqueur temporel (P_{réf 1}) marque le début ou la fin de l'envoi du signal de synchronisation ou un temps prédéterminé pendant l'envoi du signal de synchronisation.

10. Le procédé selon l'une des revendications précédentes, dans lequel à l'étape A) l'onde électrique prédéfinie détectée au moyen du contrôleur du premier dispositif correspond à l'onde P du complexe PQRS.

11. Le procédé selon l'une des revendications précédentes, dans lequel à l'étape A), l'onde électrique prédéfinie est détectée au moyen du contrôleur du premier dispositif par l'analyse d'un électrogramme, d'un électrocardiogramme, de données mesurées par un accéléromètre, de donnés mesurées par impédance cardiographie ou de donnés mesurées par un capteur acoustique.

12. Le procédé selon l'une des revendications précédentes, dans lequel sur au moins deux cycles cardiaques successifs, le premier dispositif et le deuxième dispositif communiquent entre eux de manière asynchrone pendant les étapes A et B et le moyen récepteur du deuxième dispositif est activé de manière continue pendant les au moins deux cycles cardiaques successifs.

13. Le procédé selon l'une des revendications précédentes, dans lequel le contrôleur du deuxième dispositif est configuré pour désactiver le moyen récepteur du deuxième dispositif pendant le temps restant de l'intervalle de synchronisation (108, 208) du deuxième dispositif après la réception du signal de synchronisation.

14. Le procédé selon l'une des revendications précédentes, dans lequel le contrôleur du deuxième dispositif est configuré pour envoyer un signal d'alerte par un moyen émetteur du deuxième dispositif à destination d'un moyen récepteur du premier dispositif lorsqu'un signal de synchronisation n'est pas reçu par le deuxième dispositif pendant l'intervalle de synchronisation du deuxième dispositif.

15. Système de plusieurs dispositifs médicaux implantables, comprenant au moins :
un premier dispositif comprenant au moins un moyen de détection, un moyen émetteur, et un contrôleur configuré pour analyser un signal représentatif de l'activité auriculaire, et
un deuxième dispositif, indépendant du premier dispositif médical implantable,
comprenant au moins un moyen récepteur et un contrôleur,
le moyen récepteur du deuxième dispositif étant configuré pour être activé et
désactivé par le contrôleur du deuxième dispositif,
le contrôleur du premier dispositif et le contrôleur du deuxième dispositif étant configurés pour mettre en oeuvre le procédé selon au moins une des revendications précédentes.

16. Le système de plusieurs dispositifs médicaux implantables selon la revendication 15, dans lequel
le premier dispositif médical implantable est un dispositif médical implantable sous-cutané, un enregistreur en boucle d'événement ou un stimulateur cardiaque sans sonde, et
le deuxième dispositif médical est un stimulateur cardiaque sans sonde.

## Patentansprüche

1. Verfahren zum Kommunizieren in einem System, das mehrere implantierbare medizinische Vorrichtungen umfasst, wobei eine erste Vorrichtung mindestens umfasst eine Einrichtung zum Erfassen eines für die Vorhofaktivität repräsentativen Signals, eine Übertragungseinrichtung, und eine Steuerung, die konfiguriert ist, um ein für die Vorhofaktivität repräsentatives Signal zu analysieren, und wobei eine von der ersten implantierbaren medizinischen Vorrichtung unabhängige zweite Vorrichtung mindestens eine Empfangseinrichtung und eine Steuerung umfasst, wobei das Verfahren umfasst:
(A) einen Schritt der Zeitsynchronisation der ersten Vorrichtung mit der zweiten Vorrichtung durch Senden eines Synchronisationssignals von der Sendeeinrichtung der ersten Vorrichtung an die zweite Vorrichtung, wobei das Synchronisationssignal gesendet wird, nachdem mittels der Steuerung der ersten Vorrichtung eine vordefinierte elektrische Welle des PQRS-Komplexes eines für die Vorhofaktivität repräsentativen Signals identifiziert wurde,
(B) einen Schritt der Bestimmung der Dauer eines Herzzyklus,
wobei die Schritte A und B gefolgt werden von:
(C) einem Schritt der Bestimmung eines Synchronisationsintervalls (108, 208), wobei die Dauer des Synchronisationsintervalls abhängig von der Dauer des Herzzyklus so bestimmt wird, dass sie kürzer als die Dauer des Herzzyklus ist, und wobei der Beginn des Synchronisationsintervalls (108, 208) abhängig von dem Synchronisationssignal bestimmt wird,
(D) einem Schritt der Aktivierung der Empfangseinrichtung der zweiten Vorrichtung während des Synchronisationsintervalls (108, 208), wobei die Empfangseinrichtung der zweiten Vorrichtung außerhalb des Synchronisationsintervalls (108, 208) durch die Steuerung der zweiten Vorrichtung deaktiviert wird.

2. Verfahren nach Anspruch 1, wobei für einen Herzzyklus ein das Senden des Synchronisationssignals durch die Sendeeinrichtung der ersten Vorrichtung markierender erster Zeitmarker (P_{ref1}) und ein den Empfang des Synchronisationssignals durch die Empfangseinrichtung der zweiten Vorrichtung markierender zweiter Zeitmarker (P_{ref1}) bestimmt wird, und wobei das Synchronisationsintervall (110, 210) der ersten Vorrichtung für einen nachfolgenden Herzzyklus abhängig von dem ersten Zeitmarker (P_{ref1}) bestimmt wird, wobei das Synchronisationsintervall (108, 208) der zweiten Vorrichtung für einen nachfolgenden Herzzyklus abhängig von dem zweiten Zeitmarker (P_{ref1}) bestimmt wird.

3. Verfahren nach Anspruch 2, wobei während des Synchronisationsintervalls (108, 208) der zweiten Vorrichtung:
die Empfangseinrichtung der zweiten Vorrichtung während einer Vielzahl vordefinierter Aktivierungszeitschlitze (mₒₙ) aktiviert und während einer Vielzahl vordefinierter Deaktivierungszeitschlitze (m_{off}) deaktiviert wird, wobei die Vielzahl der vordefinierten Aktivierungszeitschlitze (mₒₙ) der zweiten Vorrichtung über das Synchronisationsintervall (108, 208) der zweiten Vorrichtung so verteilt sind, dass sie mit Signalimpulszeitschlitzen der ersten Vorrichtung abhängig von dem ersten Zeitmarker (P_{ref1}) und dem zweiten Zeitmarker (P_{ref2}) synchronisiert sind.

4. Verfahren nach Anspruch 3, wobei die Übertragungseinrichtung der ersten Vorrichtung konfiguriert ist, um ein einzelnes Synchronisationssignal pro Herzzyklus zu senden.

5. Verfahren nach Anspruch 3 oder 4, wobei:
- jeder der vordefinierten Aktivierungszeitschlitze (mₒₙ) die gleiche Dauer aufweist und
- jeder der vordefinieren Deaktivierungszeitschlitze (m_{off}) die gleiche Dauer aufweist und
- jeder der vordefinierten Aktivierungs- (mₒₙ) und Deaktivierungszeitschlitze (m_{off}) im Synchronisationsintervall (108, 208) abwechselnd aufeinanderfolgt.

6. Verfahren nach Anspruch 5, wobei die Dauer eines Aktivierungszeitschlitzes (mₒₙ) kleiner oder gleich der Dauer eines Deaktivierungszeitschlitzes (m_{off}) ist.

7. Verfahren nach Anspruch 5 oder 6, wobei die Dauer eines Aktivierungszeitschlitzes (mₒₙ) zwischen 0,3 und 50 % der Dauer eines Deaktivierungszeitschlitzes (m_{off}) entspricht, insbesondere 5 bis 10%.

8. Verfahren nach Anspruch 3, wobei die Dauer eines Impulses, während dessen die Sendeeinrichtung der ersten Vorrichtung zur Ausgabe eines Synchronisationssignals konfiguriert ist, 25 bis 80 % der Dauer eines Aktivierungszeitschlitzes (mₒₙ) entspricht, insbesondere 50 %.

9. Verfahren nach Anspruch 2, wobei der erste Zeitmarker (P_{ref1}) den Beginn oder das Ende des Sendens des Synchronisationssignals oder einen vorbestimmten Zeitpunkt während des Sendens des Synchronisationssignals markiert.

10. Verfahren nach einem der vorhergehenden Ansprüche, wobei in Schritt A) die mittels der Steuerung der ersten Vorrichtung detektierte vordefinierte elektrische Welle der P-Welle des PQRS-Komplexes entspricht.

11. Verfahren nach einem der vorhergehenden Ansprüche, wobei in Schritt (A) die vordefinierte elektrische Welle mittels der Steuerung der ersten Vorrichtung durch die Analyse eines Elektrogramms, eines Elektrokardiogramms, von Messdaten eines Beschleunigungssensors, von Messdaten kardiographischer Impedanz oder akustischen Sensordaten erfasst wird.

12. Verfahren nach einem der vorhergehenden Ansprüche, wobei die erste Vorrichtung und die zweite Vorrichtung über mindestens zwei aufeinanderfolgende Herzzyklen während der Schritte A und B asynchron miteinander kommunizieren und die Empfangseinrichtung der zweiten Vorrichtung während der mindestens zwei aufeinanderfolgenden Herzzyklen kontinuierlich aktiviert wird.

13. Verfahren nach einem der vorhergehenden Ansprüche, wobei die Steuerung der zweiten Vorrichtung so konfiguriert ist, dass sie die Empfangseinrichtung der zweiten Vorrichtung während der verbleibenden Zeit des Synchronisationsintervalls (108, 208) der zweiten Vorrichtung deaktiviert, nachdem das Synchronisationssignal empfangen wurde.

14. Verfahren nach einem der vorhergehenden Ansprüche, wobei die Steuerung der zweiten Vorrichtung so konfiguriert ist, dass sie ein Warnsignal mittels einer Sendeeinrichtung der zweiten Vorrichtung an eine Empfangseinrichtung der ersten Vorrichtung sendet, falls während des Synchronisationsintervalls der zweiten Vorrichtung kein Synchronisationssignal durch die zweite Vorrichtung empfangen wird.

15. System aus mehreren implantierbaren medizinischen Vorrichtungen, mindestens umfassend:
eine erste Vorrichtung umfassend zumindest eine Erfassungseinrichtung, eine Sendeeinrichtung und eine Steuerung, die konfiguriert ist, um ein die Vorhofaktivität repräsentierendes Signal zu analysieren, und
eine zweite Vorrichtung, die von der ersten implantierbaren medizinischen Vorrichtung unabhängig ist und mindestens eine Empfangseinrichtung und eine Steuerung umfasst,
wobei die Empfangseinrichtung der zweiten Vorrichtung so konfiguriert ist, dass sie von der Steuerung der zweiten Vorrichtung ein- und ausgeschaltet wird,
wobei die Steuerung der ersten Vorrichtung und die Steuerung der zweiten Vorrichtung konfiguriert sind, um das Verfahren nach mindestens einem der vorhergehenden Ansprüche durchzuführen.

16. System aus mehreren implantierbaren medizinischen Vorrichtungen nach Anspruch 15, wobei die erste implantierbare medizinische Vorrichtung eine subkutan implantierbare medizinische Vorrichtung, ein Ereignisschleifenschreiber oder ein Herzschrittmacher ohne Sensor ist, und wobei die zweite medizinische Vorrichtung ein Herzschrittmacher ohne Sensor ist.

## Claims

1. A method of communication in a system comprising a plurality of implantable medical devices,
wherein a first device comprises at least a means for detecting a signal that is representative of atrial activity, a transmitting means, and a controller configured to analyze a signal that is representative of the atrial activity, and
a second device, which is independent of the first implantable medical device, comprising at least a receiving means and a controller,
the method comprising:
A) a step of time synchronization of the first device with the second device,
by sending a synchronization signal from the transmitting means of the first device to the second device,
the synchronization signal being sent following an identification, by means of the controller of the first device, of a predefined electrical wave of the PQRS complex of a signal representative of the atrial activity;
B) a step of determination of the duration of a cardiac cycle,
the steps A and B being followed:
C) by a step of determination of a synchronization interval (108, 208), the duration of the synchronization interval being determined based on the duration of the cardiac cycle so as to be shorter than the duration of the cardiac cycle, and the start of the synchronization interval (108, 208) being determined based on the synchronization signal,
D) by a step of activation of the receiving means of the second device during the synchronization interval (108, 208),
the receiving means of the second device being deactivated outside of the synchronization interval (108, 208) by the controller of the second device.

2. A method according to claim 1, wherein, for a cardiac cycle, a first time marker (P_{ref1}) marking the sending of the synchronization signal by means of the transmitting means of the first device is determined; and
a second time marker (P_{réf2}) marking the receiving of the synchronization signal by means of the receiving means of the second device is determined; and
the synchronization interval (110, 210) of the first device for a subsequent cardiac cycle is determined based on the first time marker (P_{réf1}), and the synchronization interval (108, 208) of the second device for a subsequent cardiac cycle is determined based on the second time marker (P_{réf2}).

3. A method according to claim 2, wherein, during the synchronization interval (108, 208) of the second device:
the receiving means of the second device is activated during a plurality of predefined activation time slots (mₒₙ) and is deactivated during a plurality of predefined deactivation time slots (m_{off}),
the plurality of predefined activation time slots (mₒₙ) of the second device being distributed over the synchronization interval (108, 208) of the second device so as to be synchronized with signal pulse slots of the first device based on the first time marker (P_{ref1}) and of the second time marker (P_{réf2}).

4. A method according to claim 3, wherein the transmitting means of the first device is configured to send a single synchronization signal per cardiac cycle.

5. A method according to any of claims 3 and 4, wherein:
- each of the predefined activation time slots (mₒₙ) is of the same duration; and
- each of the predefined deactivation time slots (m_{off}) is of the same duration; and
- each of the predefined activation time slots (mₒₙ) and the predefined deactivation time slots (m_{off}) alternatingly follow each other in the synchronization interval (108, 208).

6. A method according to claim 5, wherein the duration of an activation time slot (mₒₙ) is shorter than or equal to the duration of a deactivation time slot (m_{off}).

7. A method according to any of claims 5 and 6, wherein the duration of an activation time slot (mₒₙ) represents between 0.3% and 50% of the duration of a deactivation time slot (m_{off}), in particular between 5% and 10%.

8. A method according to claim 3, wherein the duration of a pulse during which the transmitting means of the first device is configured to transmit a synchronization signal corresponds to 25% until 80% of the duration of an activation time slot (mₒₙ), in particular to 50%.

9. A method according to claim 2, wherein the first time marker (P_{ref1}) marks the start or the end of the sending of the synchronization signal or a predetermined time during the sending of the synchronization signal.

10. A method according to any of the preceding claims, wherein, at the step A), the predefined electrical wave detected by means of the controller of the first device corresponds to a P wave of the PQRS complex.

11. A method according to any of the preceding claims, wherein, at the step A), the predefined electrical wave is detected by means of the controller of the first device using an analysis of an electrogram, an electrocardiogram, data measured by an accelerometer, data measured by cardiography impedance or data measured by an acoustic sensor.

12. A method according to any of the preceding claims, wherein, over at least two successive cardiac cycles, the first device and the second device communicate with each other in an asynchronous manner during the steps A and B, and the receiving means of the second device is continuously activated during the at least two successive cardiac cycles.

13. A method according to any of the preceding claims, wherein the controller of the second device is configured to deactivate the receiving means of the second device during the remaining time of the synchronization interval (108, 208) of the second device after the receiving of the synchronization signal.

14. A method according to any of the preceding claims, wherein the controller of the second device is configured to send an alert signal by means of a transmitting means of the second device to a receiving means of the first device when a synchronization signal is not received by the second device during the synchronization interval of the second device.

15. A system of a plurality of implantable medical devices, comprising at least:
a first device comprising at least a detecting means, a transmitting means, and a controller configured to analyze a signal that is representative of the atrial activity; and
a second device, which is independent of the first implantable medical device, comprising at least a receiving means and a controller,
the receiving means of the second device being configured to be activated and deactivated by the controller of the second device, and
the controller of the first device and the controller of the second device being configured to implement the method according to at least one of the preceding claims.

16. A system of a plurality of implantable medical devices according to claim 15, wherein:
the first implantable medical device is a subcutaneous implantable medical device, an event loop recorder, or a leadless pacemaker, and
the second medical device is a leadless pacemaker.
